(19) Europäisches Patentamt
European Patent Office
Office européen des brevets

(11) EP 4 324 341 B1

(12) EUROPEAN PATENT SPECIFICATION

(45) Date of publication and mention of the grant of the patent:
**09.07.2025 Bulletin 2025/28**

(21) Application number: **24150767.2**

(22) Date of filing: **31.10.2019**

(51) International Patent Classification (IPC):
***A24B 15/167*** *(2020.01)* ***A24F 40/10*** *(2020.01)*
***A61K 31/465*** *(2006.01)* ***A24B 15/28*** *(2006.01)*
***A24B 15/30*** *(2006.01)*

(52) Cooperative Patent Classification (CPC):
**A24B 15/167; A24B 15/281; A24B 15/283; A24B 15/30; A61K 31/465;** A24F 40/10; Y02E 60/10

(54) **AEROSOLISABLE FORMULATION**

AEROSOLIERBARE FORMULIERUNG

FORMULATION AÉROSOLISABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2018 GB 201817866**
**03.05.2019 GB 201906242**

(43) Date of publication of application:
**21.02.2024 Bulletin 2024/08**

(62) Document number(s) of the earlier application(s) in accordance with Art. 76 EPC:
**19798369.5 / 3 873 249**

(73) Proprietor: **Nicoventures Trading Limited**
**London**
**Greater London WC2R 3LA (GB)**

(72) Inventor: **CABOT, Ross**
**London, WC2R 3LA (GB)**

(74) Representative: **D Young & Co LLP**
**3 Noble Street**
**London EC2V 7BQ (GB)**

(56) References cited:
**WO-A1-96/04937**
**US-A1- 2008 138 399**
**US-A1- 2016 198 759**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 324 341 B1

## Description

### FIELD OF THE INVENTION

The present disclosure relates to an aerosolisable formulation, a method of forming the same, a container containing the same, a device containing the same and processes and uses of the same.

### BACKGROUND TO THE INVENTION

Electronic aerosol provision systems such as e-cigarettes generally contain a reservoir of liquid which is to be vaporised, typically containing nicotine. When a user inhales on the device, a heater is activated to vaporise a small amount of liquid, which is therefore inhaled by the user.

US 2016/198759 A1 discloses an electronic aerosol provision system comprising an improved vaping fluid.

The use of e-cigarettes in the UK has grown rapidly, and it has been estimated that there are now over a million people using them in the UK.

One challenge faced in providing such systems is to provide from the aerosol provision device an aerosol to be inhaled which provides consumers with an acceptable experience. Some consumers may prefer an e-cigarette that generates an aerosol that closely 'mimics' smoke inhaled from a tobacco product such as a cigarette. Aerosols from e-cigarettes and smoke from tobacco products such as cigarettes provides to the user a complex chain of flavour in the mouth, nicotine absorption in the mouth and throat, followed by nicotine absorption in the lungs. These various aspects are described by users in terms of flavour, intensity/quality, impact, irritation/smoothness and nicotine reward. Nicotine contributes to a number of these factors, and is strongly associated with factors such as impact, irritation and smoothness; these are readily perceived by consumers, and e-cigarettes may offer too much or too little of these parameters for consumers, depending upon individual preferences. Nicotine reward is particularly complex as it results from both the amount of and speed with which nicotine is absorbed from the lining of the mouth, this is typically nicotine in the vapour phase, and from the amount and speed nicotine that is absorbed from the lungs, this is typically nicotine in the particulate phase of the aerosol which is inhaled. Each of these factors, and their balance, can strongly contribute to consumer acceptability of an e-cigarette. Providing means to optimise the overall vaping experience is therefore desirable to e-cigarette manufacturers.

A further challenge facing such systems is the continued demand for harm reduction. Harm from cigarette and e-cigarette devices primarily comes from toxicants. Therefore, there is a desire to reduce or remove the components which may form toxicants.

### SUMMARY OF THE INVENTION

The invention is as defined in the appended claims. In one aspect there is provided an aerosolisable formulation comprising

(i) water
(ii) one or more flavours to be encapsulated;
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof; and
(iv) nicotine;

wherein in the presence of water the energy of binding of the one or more encapsulating materials with the one or more flavours to be encapsulated is from -0.5 to -8 kcal/mol.

In one aspect there is provided an aerosolisable formulation comprising

(i) water
(ii) one or more flavours to be encapsulated; and
(iii) one or more encapsulating materials having a solubility in water of at least 50% of the solubility in water of the one or more flavours to be encapsulated wherein the one or more encapsulating material are selected from the group consisting of substituted ($\alpha$)-cyclodextrin, substituted ($\beta$)-cyclodextrin, substituted ($\gamma$)-cyclodextrin, and mixtures thereof; and
(iv) nicotine.

In one aspect there is provided a process for forming an aerosol, the process comprising aerosolising an aerosolisable formulation comprising

(i) water
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

In one aspect there is provided a contained aerosolisable formulation comprising

(a) a container; and
(b) an aerosolisable formulation comprising

(i) water
(ii) one or more flavours to be encapsulated;
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof; and
(iv) nicotine;

wherein in the presence of water the energy of binding of the one or more encapsulating materials with the one or more flavours to be encapsulated is from -0.5 to -8 kcal/mol.

In one aspect there is provided an electronic aerosol provision system comprising:

(a) an aerosoliser for aerosolising formulation for inhalation by a user of the electronic aerosol provision system;
(b) a power supply comprising a cell or battery for supplying power to the aerosoliser
(c) an aerosolisable formulation comprising

(i) water
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

In one aspect there is provided a process for improving the sensory properties of an aerosolised formulation, the process comprising the steps of aerosolising an aerosolisable formulation comprising

(i) water;
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

## DETAILED DESCRIPTION

As discussed herein in one aspect there is provided an aerosolisable formulation comprising (i) water; (ii) one or more flavours to be encapsulated; (iii) one or more encapsulating materials selected from the group consisting of substituted ($\alpha$)-cyclodextrin, substituted ($\beta$)-cyclodextrin, substituted ($\gamma$)-cyclodextrin, and mixtures thereof; and (iv) nicotine; wherein in the presence of water the energy of binding of the one or more encapsulating materials with the one or more flavours to be encapsulated is from -0.5 to -8 kcal/mol.

We have found that an advantageous system may be provided in which, in the presence of water, the energy of binding of the one or more encapsulating materials with the one or more flavours to be encapsulated is from -0.5 to -8 kcal/mol. We have found that by selection of one or more encapsulating materials together with selection of the one or more flavours to be encapsulated such that the two materials have the required energy of binding an advantageous flavour delivery system is provided. In particular, the flavour delivery system binds the flavour strongly enough for it to be delivered in use but not so strongly that it will not dissociate in use from the encapsulating material. Thus a flavour may be stably delivered whilst still providing a strong flavour release for the end user.

As is understood by one skilled in the art, nicotine may exist in unprotonated form, monoprotonated form or diprotonated form. The structures of each of these forms are given below.

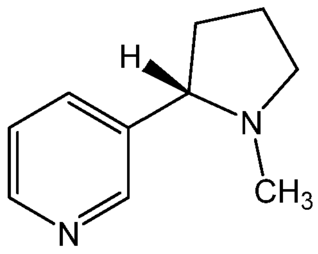

Unprotonated nicotine

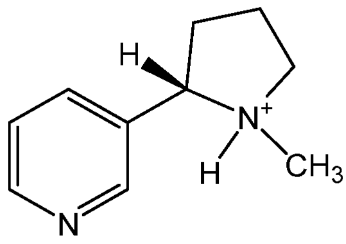

monoprotonated nicotine

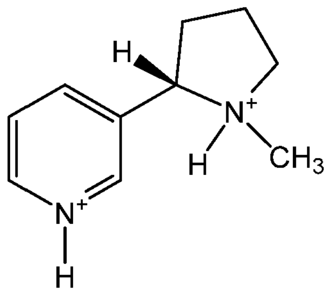

diprotonated nicotine

Reference in the specification to protonated form means both monoprotonated nicotine and diprotonated nicotine. Reference in the specification to amounts in the protonated form means the combined amount of monoprotonated nicotine and diprotonated nicotine. Furthermore, when reference is made to a fully protonated formulation it will be understood that at any one time there may be very minor amounts of unprotonated nicotine present, e.g. less than 1% unprotonated.

For ease of reference, these and further aspects of the present invention are now discussed under appropriate section headings. However, the teachings under each section are not necessarily limited to each particular section.

*Energy of Binding*

As discussed herein, in the presence of water the energy of binding of the one or more encapsulating materials with the one or more flavours to be encapsulated is from -0.5 to -8 kcal/mol.

In one aspect the energy of binding may be determined by molecular modelling and, in particular, using docking performed in Autodock 4.2 [The Scripps Research Institute, La Jolla, CA, USA] (Morris, G. M., Huey, R., Lindstrom, W., Sanner, M. F., Belew, R. K., Goodsell, D. S., & Olson, A. J. (2009). Autodock4 and AutodockTools4: automated docking with selective receptor flexibility. J. Computational Chemistry, 2785-2791). The docking performed in Autodock 4.2 may be performed using the following settings:
Grid Point Spacing (Angstroms): 0.375
Number of grid points in each Cartesian direction

| | |
|---|---|
| x: | 40 |
| y: | 40 |
| z: | 40 |

User-specified initial position for ligand: random
Initial relative dihedral offset: random
User-specified initial relative dihedrals: random
Docking search parameter: Genetic Algorithm
Number of requested GA dockings: 10 runs
Population size: 150
Maximum number of evaluations: 2500000
Maximum number of top individuals that automatically survive: 1
Rate of gene mutation: 0.02
Rate of crossover: 0.08
GA crossover mode: "twopt"
Mean of Cauchy distribution for gene mutation(alpha parameter): 0
Variance of Cauchy distribution for gene mutation(beta parameter): 1
Number of generations fro picking worst individuals : 10
Docking output: Lamarckian GA

In one aspect, in the presence of water the energy of binding of the one or more encapsulating materials with the one or more flavours to be encapsulated is from -1 to -8 kcal/mol. In one aspect, in the presence of water the energy of binding of the one or more encapsulating materials with the one or more flavours to be encapsulated is from -1.5 to -8

kcal/mol. In one aspect, in the presence of water the energy of binding of the one or more encapsulating materials with the one or more flavours to be encapsulated is from -2 to -8 kcal/mol. In one aspect, in the presence of water the energy of binding of the one or more encapsulating materials with the one or more flavours to be encapsulated is from -2.5 to -8 kcal/mol. In one aspect, in the presence of water the energy of binding of the one or more encapsulating materials with the one or more flavours to be encapsulated is from -3 to -8 kcal/mol. In one aspect, in the presence of water the energy of binding of the one or more encapsulating materials with the one or more flavours to be encapsulated is from -3.5 to -8 kcal/mol. In one aspect, in the presence of water the energy of binding of the one or more encapsulating materials with the one or more flavours to be encapsulated is from -4 to -8 kcal/mol. In one aspect, in the presence of water the energy of binding of the one or more encapsulating materials with the one or more flavours to be encapsulated is from -4.5 to -8 kcal/mol. In one aspect, in the presence of water the energy of binding of the one or more encapsulating materials with the one or more flavours to be encapsulated is from -5 to -8 kcal/mol. In one aspect, in the presence of water the energy of binding of the one or more encapsulating materials with the one or more flavours to be encapsulated is from -5 to -7.5 kcal/mol. In one aspect, in the presence of water the energy of binding of the one or more encapsulating materials with the one or more flavours to be encapsulated is from -5 to -7 kcal/mol.

In one aspect, in the presence of water the energy of binding of the one or more encapsulating materials with the one or more flavours to be encapsulated is from -0.5 to -7.5 kcal/mol. In one aspect, in the presence of water the energy of binding of the one or more encapsulating materials with the one or more flavours to be encapsulated is from -0.5 to -7 kcal/mol. In one aspect, in the presence of water the energy of binding of the one or more encapsulating materials with the one or more flavours to be encapsulated is from -0.5 to -6.5 kcal/mol. In one aspect, in the presence of water the energy of binding of the one or more encapsulating materials with the one or more flavours to be encapsulated is from -0.5 to -6 kcal/mol. In one aspect, in the presence of water the energy of binding of the one or more encapsulating materials with the one or more flavours to be encapsulated is from -1 to -6 kcal/mol. In one aspect, in the presence of water the energy of binding of the one or more encapsulating materials with the one or more flavours to be encapsulated is from -1.5 to -6 kcal/mol. In one aspect, in the presence of water the energy of binding of the one or more encapsulating materials with the one or more flavours to be encapsulated is from -2 to -6 kcal/mol. In one aspect, in the presence of water the energy of binding of the one or more encapsulating materials with the one or more flavours to be encapsulated is from -2.5 to -6 kcal/mol. In one aspect, in the presence of water the energy of binding of the one or more encapsulating materials with the one or more flavours to be encapsulated is from -3 to -6 kcal/mol. In one aspect, in the presence of water the energy of binding of the one or more encapsulating materials with the one or more flavours to be encapsulated is from -3.5 to -6 kcal/mol. In one aspect, in the presence of water the energy of binding of the one or more encapsulating materials with the one or more flavours to be encapsulated is from -4 to -6 kcal/mol. In one aspect, in the presence of water the energy of binding of the one or more encapsulating materials with the one or more flavours to be encapsulated is from -4.5 to -6 kcal/mol. In one aspect, in the presence of water the energy of binding of the one or more encapsulating materials with the one or more flavours to be encapsulated is from -5 to -6 kcal/mol.

*Water*

As discussed herein the aerosolisable formulation contains water. In one aspect water is present in an amount of at least 30 wt.% based on the aerosolisable formulation. In one aspect water is present in an amount of at least 35 wt.% based on the aerosolisable formulation. In one aspect water is present in an amount of at least 40 wt.% based on the aerosolisable formulation. In one aspect water is present in an amount of at least 45 wt.% based on the aerosolisable formulation. In one aspect water is present in an amount of at least 50 wt.% based on the aerosolisable formulation. In one aspect water is present in an amount of at least 55 wt.% based on the aerosolisable formulation. In one aspect water is present in an amount of at least 60 wt.% based on the aerosolisable formulation. In one aspect water is present in an amount of at least 65 wt.% based on the aerosolisable formulation. In one aspect water is present in an amount of at least 70 wt.% based on the aerosolisable formulation. In one aspect water is present in an amount of at least 75 wt.% based on the aerosolisable formulation. In one aspect water is present in an amount of at least 80 wt.% based on the aerosolisable formulation. In one aspect water is present in an amount of at least 85 wt.% based on the aerosolisable formulation. In one aspect water is present in an amount of at least 90 wt.% based on the aerosolisable formulation. In one aspect water is present in an amount of at least 95 wt.% based on the aerosolisable formulation. In one aspect water is present in an amount of at least 99 wt.% based on the aerosolisable formulation.

In one aspect water is present in an amount of from 30 to 99 wt.% based on the aerosolisable formulation. In one aspect water is present in an amount of from 35 to 99 wt.% based on the aerosolisable formulation. In one aspect water is present in an amount of from 40 to 99 wt.% based on the aerosolisable formulation. In one aspect water is present in an amount of from 45 to 99 wt.% based on the aerosolisable formulation. In one aspect water is present in an amount of from 50 to 99 wt.% based on the aerosolisable formulation. In one aspect water is present in an amount of from 55 to 99 wt.% based on the aerosolisable formulation. In one aspect water is present in an amount of from 60 to 99 wt.% based on the aerosolisable formulation. In one aspect water is present in an amount of from 65 to 99 wt.% based on the aerosolisable

formulation. In one aspect water is present in an amount of from 70 to 99 wt.% based on the aerosolisable formulation. In one aspect water is present in an amount of from 75 to 99 wt.% based on the aerosolisable formulation. In one aspect water is present in an amount of from 80 to 99 wt.% based on the aerosolisable formulation. In one aspect water is present in an amount of from 85 to 99 wt.% based on the aerosolisable formulation. In one aspect water is present in an amount of from 90 to 99 wt.% based on the aerosolisable formulation. In one aspect water is present in an amount of from 95 to 99 wt.% based on the aerosolisable formulation.

The use of water allows for the replacement of some or all of the glycerol, propylene glycol, 1,3-propane diol and mixtures thereof typically used in e-cigarettes. In one aspect the aerosolisable formulation contains glycerol, propylene glycol, 1,3-propane diol and mixtures thereof in a combined amount of no greater than 10 wt.% based on the aerosolisable formulation. In one aspect the aerosolisable formulation contains glycerol, propylene glycol, 1,3-propane diol and mixtures thereof in a combined amount of no greater than 8 wt.% based on the aerosolisable formulation. In one aspect the aerosolisable formulation contains glycerol, propylene glycol, 1,3-propane diol and mixtures thereof in a combined amount of no greater than 5 wt.% based on the aerosolisable formulation. In one aspect the aerosolisable formulation contains glycerol, propylene glycol, 1,3-propane diol and mixtures thereof in a combined amount of no greater than 2 wt.% based on the aerosolisable formulation. In one aspect the aerosolisable formulation contains glycerol, propylene glycol, 1,3-propane diol and mixtures thereof in a combined amount of no greater than 1 wt.% based on the aerosolisable formulation. In one aspect the aerosolisable formulation contains glycerol, propylene glycol, 1,3-propane diol and mixtures thereof in a combined amount of no greater than 0.5 wt.% based on the aerosolisable formulation. In one aspect the aerosolisable formulation contains glycerol, propylene glycol, 1,3-propane diol and mixtures thereof in a combined amount of no greater than 0.2 wt.% based on the aerosolisable formulation. In one aspect the aerosolisable formulation contains glycerol, propylene glycol, 1,3-propane diol and mixtures thereof in a combined amount of no greater than 0.1 wt.% based on the aerosolisable formulation. In one aspect the aerosolisable formulation contains glycerol, propylene glycol, 1,3-propane diol and mixtures thereof in a combined amount of no greater than 0.01 wt.% based on the aerosolisable formulation. In one aspect the aerosolisable formulation contains no glycerol, propylene glycol, 1,3-propane diol and mixtures thereof.

In one aspect the aerosolisable formulation contains glycerol, propylene glycol, and mixtures thereof in a combined amount of no greater than 10 wt.% based on the aerosolisable formulation. In one aspect the aerosolisable formulation contains glycerol, propylene glycol, and mixtures thereof in a combined amount of no greater than 8 wt.% based on the aerosolisable formulation. In one aspect the aerosolisable formulation contains glycerol, propylene glycol, and mixtures thereof in a combined amount of no greater than 5 wt.% based on the aerosolisable formulation. In one aspect the aerosolisable formulation contains glycerol, propylene glycol, and mixtures thereof in a combined amount of no greater than 2 wt.% based on the aerosolisable formulation. In one aspect the aerosolisable formulation contains glycerol, propylene glycol, and mixtures thereof in a combined amount of no greater than 1 wt.% based on the aerosolisable formulation. In one aspect the aerosolisable formulation contains glycerol, propylene glycol, and mixtures thereof in a combined amount of no greater than 0.5 wt.% based on the aerosolisable formulation. In one aspect the aerosolisable formulation contains glycerol, propylene glycol, and mixtures thereof in a combined amount of no greater than 0.2 wt.% based on the aerosolisable formulation. In one aspect the aerosolisable formulation contains glycerol, propylene glycol, and mixtures thereof in a combined amount of no greater than 0.1 wt.% based on the aerosolisable formulation. In one aspect the aerosolisable formulation contains glycerol, propylene glycol, and mixtures thereof in a combined amount of no greater than 0.01 wt.% based on the aerosolisable formulation. In one aspect the aerosolisable formulation contains no glycerol, propylene glycol, and mixtures thereof.

In one aspect the aerosolisable formulation contains glycerol in an amount of no greater than 10 wt.% based on the aerosolisable formulation. In one aspect the aerosolisable formulation contains glycerol in an amount of no greater than 8 wt.% based on the aerosolisable formulation. In one aspect the aerosolisable formulation contains glycerol in an amount of no greater than 5 wt.% based on the aerosolisable formulation. In one aspect the aerosolisable formulation contains glycerol in an amount of no greater than 2 wt.% based on the aerosolisable formulation. In one aspect the aerosolisable formulation contains glycerol in an amount of no greater than 1 wt.% based on the aerosolisable formulation. In one aspect the aerosolisable formulation contains glycerol in an amount of no greater than 0.5 wt.% based on the aerosolisable formulation. In one aspect the aerosolisable formulation contains glycerol in an amount of no greater than 0.2 wt.% based on the aerosolisable formulation. In one aspect the aerosolisable formulation contains glycerol in an amount of no greater than 0.1 wt.% based on the aerosolisable formulation. In one aspect the aerosolisable formulation contains glycerol in an amount of no greater than 0.01 wt.% based on the aerosolisable formulation. In one aspect the aerosolisable formulation contains no glycerol.

In one aspect the aerosolisable formulation contains propylene glycol in an amount of no greater than 10 wt.% based on the aerosolisable formulation. In one aspect the aerosolisable formulation contains propylene glycol in an amount of no greater than 8 wt.% based on the aerosolisable formulation. In one aspect the aerosolisable formulation contains propylene glycol in an amount of no greater than 5 wt.% based on the aerosolisable formulation. In one aspect the aerosolisable formulation contains propylene glycol in an amount of no greater than 2 wt.% based on the aerosolisable

formulation. In one aspect the aerosolisable formulation contains propylene glycol in an amount of no greater than 1 wt.% based on the aerosolisable formulation. In one aspect the aerosolisable formulation contains propylene glycol in an amount of no greater than 0.5 wt.% based on the aerosolisable formulation. In one aspect the aerosolisable formulation contains propylene glycol in an amount of no greater than 0.2 wt.% based on the aerosolisable formulation. In one aspect the aerosolisable formulation contains propylene glycol in an amount of no greater than 0.1 wt.% based on the aerosolisable formulation. In one aspect the aerosolisable formulation contains propylene glycol in an amount of no greater than 0.01 wt.% based on the aerosolisable formulation. In one aspect the aerosolisable formulation contains no propylene glycol.

*Nicotine*

Nicotine formulations may be provided having desirable properties of flavour, impact, irritation, smoothness and/or nicotine reward for the user. In one aspect nicotine is present in an amount of no greater than 6 wt% based on the total weight of the aerosolisable formulation. In one aspect nicotine is present in an amount of from 0.01 to 6 wt% based on the total weight of the aerosolisable formulation. In one aspect nicotine is present in an amount of from 0.02 to 6 wt% based on the total weight of the aerosolisable formulation. In one aspect nicotine is present in an amount of from 0.05 to 6 wt% based on the total weight of the aerosolisable formulation. In one aspect nicotine is present in an amount of from 0.08 to 6 wt% based on the total weight of the aerosolisable formulation. In one aspect nicotine is present in an amount of from 0.01 to 5 wt% based on the total weight of the aerosolisable formulation. In one aspect nicotine is present in an amount of from 0.02 to 5 wt% based on the total weight of the aerosolisable formulation. In one aspect nicotine is present in an amount of from 0.05 to 5 wt% based on the total weight of the aerosolisable formulation. In one aspect nicotine is present in an amount of from 0.08 to 5 wt% based on the total weight of the aerosolisable formulation. In one aspect nicotine is present in an amount of no greater than 4 wt% based on the total weight of the aerosolisable formulation. In one aspect nicotine is present in an amount of from 0.01 to 4 wt% based on the total weight of the aerosolisable formulation. In one aspect nicotine is present in an amount of from 0.02 to 4 wt% based on the total weight of the aerosolisable formulation. In one aspect nicotine is present in an amount of from 0.05 to 4 wt% based on the total weight of the aerosolisable formulation. In one aspect nicotine is present in an amount of from 0.08 to 4 wt% based on the total weight of the aerosolisable formulation. In one aspect nicotine is present in an amount of no greater than 3 wt% based on the total weight of the aerosolisable formulation. In one aspect nicotine is present in an amount of from 0.01 to 3 wt% based on the total weight of the aerosolisable formulation. In one aspect nicotine is present in an amount of from 0.02 to 3 wt% based on the total weight of the aerosolisable formulation. In one aspect nicotine is present in an amount of from 0.05 to 3 wt% based on the total weight of the aerosolisable formulation. In one aspect nicotine is present in an amount of from 0.08 to 3 wt% based on the total weight of the aerosolisable formulation. In one aspect nicotine is present in an amount of no greater than 2 wt% based on the total weight of the aerosolisable formulation. In one aspect nicotine is present in an amount of from 0.01 to 2 wt% based on the total weight of the aerosolisable formulation. In one aspect nicotine is present in an amount of from 0.02 to 2 wt% based on the total weight of the aerosolisable formulation. In one aspect nicotine is present in an amount of from 0.05 to 2 wt% based on the total weight of the aerosolisable formulation. In one aspect nicotine is present in an amount of from 0.08 to 2 wt% based on the total weight of the aerosolisable formulation. In one aspect nicotine is present in an amount of no greater than 1 wt% based on the total weight of the aerosolisable formulation. In one aspect nicotine is present in an amount of from 0.01 to 1 wt% based on the total weight of the aerosolisable formulation. In one aspect nicotine is present in an amount of from 0.02 to 1 wt% based on the total weight of the aerosolisable formulation. In one aspect nicotine is present in an amount of from 0.05 to 1 wt% based on the total weight of the aerosolisable formulation. In one aspect nicotine is present in an amount of from 0.08 to 1 wt% based on the total weight of the aerosolisable formulation. In one aspect nicotine is present in an amount of from 0.1 to 1 wt% based on the total weight of the aerosolisable formulation. In one aspect nicotine is present in an amount of no greater than 0.6 wt% based on the total weight of the aerosolisable formulation. In one aspect nicotine is present in an amount of from 0.01 to 0.6 wt% based on the total weight of the aerosolisable formulation. In one aspect nicotine is present in an amount of from 0.02 to 0.6 wt% based on the total weight of the aerosolisable formulation. In one aspect nicotine is present in an amount of from 0.05 to 0.6 wt% based on the total weight of the aerosolisable formulation. In one aspect nicotine is present in an amount of from 0.08 to 0.6 wt% based on the total weight of the aerosolisable formulation. In one aspect nicotine is present in an amount of from 0.1 to 0.6 wt% based on the total weight of the aerosolisable formulation. In one aspect nicotine is present in an amount of no greater than 0.5 wt% based on the total weight of the aerosolisable formulation. In one aspect nicotine is present in an amount of from 0.01 to 0.5 wt% based on the total weight of the aerosolisable formulation. In one aspect nicotine is present in an amount of from 0.02 to 0.5 wt% based on the total weight of the aerosolisable formulation. In one aspect nicotine is present in an amount of from 0.05 to 0.5 wt% based on the total weight of the aerosolisable formulation. In one aspect nicotine is present in an amount of from 0.08 to 0.5 wt% based on the total weight of the aerosolisable formulation. In one aspect nicotine is present in an amount of no greater than 0.2 wt% based on the total weight of the aerosolisable formulation. In one aspect nicotine is present in an amount of from 0.01 to 0.2 wt% based on the total weight of the aerosolisable formulation. In one aspect

nicotine is present in an amount of from 0.02 to 0.2 wt% based on the total weight of the aerosolisable formulation. In one aspect nicotine is present in an amount of from 0.05 to 0.2 wt% based on the total weight of the aerosolisable formulation. In one aspect nicotine is present in an amount of from 0.08 to 0.2 wt% based on the total weight of the aerosolisable formulation. In one aspect nicotine is present in an amount of no greater than 0.1 wt% based on the total weight of the aerosolisable formulation. In one aspect nicotine is present in an amount of from 0.01 to 0.1 wt% based on the total weight of the aerosolisable formulation. In one aspect nicotine is present in an amount of from 0.02 to 0.1 wt% based on the total weight of the aerosolisable formulation. In one aspect nicotine is present in an amount of from 0.05 to 0.1 wt% based on the total weight of the aerosolisable formulation. In one aspect nicotine is present in an amount of from 0.08 to 0.1 wt% based on the total weight of the aerosolisable formulation.

The formulation may comprise nicotine in protonated form. The formulation may comprise nicotine in unprotonated form. In one aspect the formulation comprises nicotine in unprotonated form and nicotine in monoprotonated form. In one aspect the formulation comprises nicotine in unprotonated form and nicotine in diprotonated form. In one aspect the formulation comprises nicotine in unprotonated form, nicotine in monoprotonated form and nicotine in diprotonated form.

In one aspect at least 5wt% of the nicotine present in the formulation is in protonated form. In one aspect at least 10wt% of the nicotine present in the formulation is in protonated form. In one aspect at least 15wt% of the nicotine present in the formulation is in protonated form. In one aspect at least 20wt% of the nicotine present in the formulation is in protonated form. In one aspect at least 25wt% of the nicotine present in the formulation is in protonated form. In one aspect at least 30wt% of the nicotine present in the formulation is in protonated form. In one aspect at least 35wt% of the nicotine present in the formulation is in protonated form. In one aspect at least 40wt% of the nicotine present in the formulation is in protonated form. In one aspect at least 45wt% of the nicotine present in the formulation is in protonated form. In one aspect at least 50wt% of the nicotine present in the formulation is in protonated form. In one aspect at least 55wt% of the nicotine present in the formulation is in protonated form. In one aspect at least 60wt% of the nicotine present in the formulation is in protonated form. In one aspect at least 65wt% of the nicotine present in the formulation is in protonated form. In one aspect at least 70wt% of the nicotine present in the formulation is in protonated form. In one aspect at least 75wt% of the nicotine present in the formulation is in protonated form. In one aspect at least 80wt% of the nicotine present in the formulation is in protonated form. In one aspect at least 85wt% of the nicotine present in the formulation is in protonated form. In one aspect at least 90wt% of the nicotine present in the formulation is in protonated form. In one aspect at least 95wt% of the nicotine present in the formulation is in protonated form. In one aspect at least 99wt% of the nicotine present in the formulation is in protonated form. In one aspect at least 99.9wt% of the nicotine present in the formulation is in protonated form.

In one aspect from 50 to 95 wt% of the nicotine present in the formulation is in protonated form. In one aspect from 55 to 95 wt% of the nicotine present in the formulation is in protonated form. In one aspect from 60 to 95 wt% of the nicotine present in the formulation is in protonated form. In one aspect from 65 to 95 wt% of the nicotine present in the formulation is in protonated form. In one aspect from 70 to 95 wt% of the nicotine present in the formulation is in protonated form. In one aspect from 75 to 95 wt% of the nicotine present in the formulation is in protonated form. In one aspect from 80 to 95 wt% of the nicotine present in the formulation is in protonated form. In one aspect from 85 to 95 wt% of the nicotine present in the formulation is in protonated form. In one aspect from 90 to 95 wt% of the nicotine present in the formulation is in protonated form.

In one aspect from 50 to 99 wt% of the nicotine present in the formulation is in protonated form. In one aspect from 55 to 99 wt% of the nicotine present in the formulation is in protonated form. In one aspect from 60 to 99 wt% of the nicotine present in the formulation is in protonated form. In one aspect from 65 to 99 wt% of the nicotine present in the formulation is in protonated form. In one aspect from 70 to 99 wt% of the nicotine present in the formulation is in protonated form. In one aspect from 75 to 99 wt% of the nicotine present in the formulation is in protonated form. In one aspect from 80 to 99 wt% of the nicotine present in the formulation is in protonated form. In one aspect from 85 to 99 wt% of the nicotine present in the formulation is in protonated form. In one aspect from 90 to 99 wt% of the nicotine present in the formulation is in protonated form.

The relevant amounts of nicotine which are present in the formulation in protonated form are specified herein. These amounts may be readily calculated by one skilled in the art. Nicotine, 3-(1-methylpyrrolidin-2-yl) pyridine, is a diprotic base with pKa of 3.12 for the pyridine ring and 8.02 for the pyrrolidine ring It can exist in pH-dependent protonated (mono- and di-) and non-protonated (free base) forms which have different bioavailability.

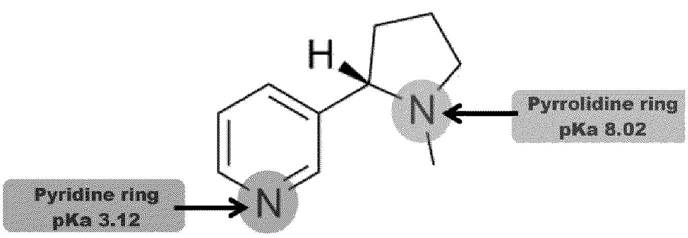

The distribution of protonated and non-protonated nicotine will vary at various pH increments.

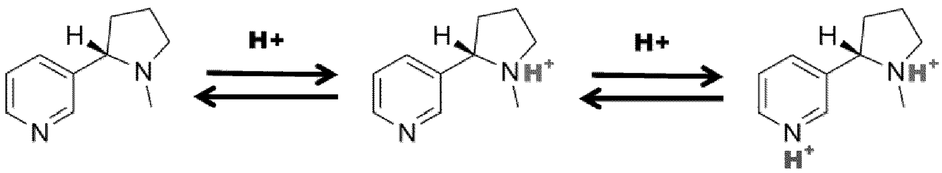

The fraction of non-protonated nicotine will be predominant at high pH levels whilst a decrease in the pH will see an increase of the fraction of protonated nicotine (mono- or di- depending on the pH). If the relative fraction of protonated nicotine and the total amount of nicotine in the sample are known, the absolute amount of protonated nicotine can be calculated.

The relative fraction of protonated nicotine in formulation can be calculated by using the Henderson-Hasselbalch equation, which describes the pH as a derivation of the acid dissociation constant equation, and it is extensively employed in chemical and biological systems. Consider the following equilibrium:

$$B + H^+ \rightleftarrows BH^+$$

The Henderson-Hasselbalch equation for this equilibrium is:

$$pH = pKa + \log\frac{[B]}{[BH+]}$$

Where [B] is the amount of non-protonated nicotine (i.e. free base), [BH+] the amount of protonated nicotine (i.e. conjugate acid) and pKa is the reference pKa value for the pyrrolidine ring nitrogen of nicotine (pKa=8.02). The relative fraction of protonated nicotine can be derived from the alpha value of the non-protonated nicotine calculated from the Henderson-Hasselbalch equation as:

$$\%\,protonated\ nicotine = 100 - \left\{\frac{\frac{[B]}{[BH+]}}{\left\{1 + \frac{[B]}{[BH+]}\right\}} * 100\right\}$$

Determination of pKa values of nicotine formulations was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, .Anal. Methods, 2013,5, 81-88.

*Acid*

In one aspect the aerosolisable formulation further comprises an acid. The acid may be any suitable acid. In one aspect the acid is an organic acid. In one aspect the acid is a carboxylic acid. In one aspect the acid is an organic carboxylic acid.

In one aspect the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof. In one aspect the acid is selected from the group consisting of citric acid, benzoic acid, levulinic acid, lactic acid, sorbic acid, and mixtures thereof. In one aspect the acid is selected from the group consisting of citric acid, benzoic acid, levulinic acid, and mixtures thereof. In one aspect the acid is at least citric acid. In one aspect the acid consists of citric acid.

In one aspect the acid is selected from acids having a pka of from 2 to 5. In one aspect the acid is a weak acid. In one aspect the acid is a weak organic acid.

In one aspect the acid has a solubility in water of at least 2g/L at 20 °C. In one aspect the acid has a solubility in water of at least 5g/L at 20 °C. In one aspect the acid has a solubility in water of at least 10g/L at 20 °C. In one aspect the acid has a solubility in water of at least 20g/L at 20 °C. In one aspect the acid has a solubility in water of at least 50g/L at 20 °C. In one aspect the acid has a solubility in water of at least 100g/L at 20 °C. In one aspect the acid has a solubility in water of at least 200g/L at 20 °C. In one aspect the acid has a solubility in water of at least 300g/L at 20 °C. In one aspect the acid has a

solubility in water of at least 400g/L at 20 °C. In one aspect the acid has a solubility in water of at least 500g/L at 20 °C. In one aspect the acid has a solubility in water of at least 600g/L at 20 °C. In one aspect the acid has a solubility in water of at least 700g/L at 20 °C. In one aspect the acid has a solubility in water of at least 800g/L at 20 °C. In one aspect the acid has a solubility in water of at least 900g/L at 20 °C. In one aspect the acid has a solubility in water of at least 1000g/L at 20 °C. In one aspect the acid has a solubility in water of at least 1100g/L at 20°C.

The molar ratio of acid to nicotine may be selected as desired. In one aspect the molar ratio of acid to nicotine is from 5:1 to 1:5. In one aspect the molar ratio of acid to nicotine is from 4:1 to 1:4. In one aspect the molar ratio of acid to nicotine is from 3:1 to 1:3. In one aspect the molar ratio of acid to nicotine is from 2:1 to 1:2. In one aspect the molar ratio of acid to nicotine is from 1.5:1 to 1:1.5. In one aspect the molar ratio of acid to nicotine is from 1.2:1 to 1:1.2. In one aspect the molar ratio of acid to nicotine is from 5:1 to 1:1. In one aspect the molar ratio of acid to nicotine is from 4:1 to 1:1. In one aspect the molar ratio of acid to nicotine is from 3:1 to 1:1. In one aspect the molar ratio of acid to nicotine is from 2:1 to 1:1. In one aspect the molar ratio of acid to nicotine is from 1.5:1 to 1:1. In one aspect the molar ratio of acid to nicotine is from 1.2:1 to 1:1.

In one aspect the total content of acid present in the formulation is no greater than 5 mole equivalents based on the nicotine. In one aspect the total content of acid present in the formulation is no greater than 4 mole equivalents based on the nicotine. In one aspect the total content of acid present in the formulation is no greater than 3 mole equivalents based on the nicotine. In one aspect the total content of acid present in the formulation is no greater than 2 mole equivalents based on the nicotine. In one aspect the total content of acid present in the formulation is no greater than 1 mole equivalents based on the nicotine.

In one aspect the total content of acid present in the formulation is no less than 0.01 mole equivalents based on the nicotine. In one aspect the total content of acid present in the formulation is no less than 0.05 mole equivalents based on the nicotine. In one aspect the total content of acid present in the formulation is no less than 0.1 mole equivalents based on the nicotine. In one aspect the total content of acid present in the formulation is no less than 0.2 mole equivalents based on the nicotine. In one aspect the total content of acid present in the formulation is no less than 0.3 mole equivalents based on the nicotine. In one aspect the total content of acid present in the formulation is no less than 0.4 mole equivalents based on the nicotine. In one aspect the total content of acid present in the formulation is no less than 0.5 mole equivalents based on the nicotine. In one aspect the total content of acid present in the formulation is no less than 0.7 mole equivalents based on the nicotine.

The acid may be present in any suitable amount. In one aspect the acid is present in an amount of no greater than 6 wt% based on the aerosolisable formulation. In one aspect the acid is present in an amount of from 0.01 to 6 wt% based on the aerosolisable formulation. In one aspect the acid is present in an amount of from 0.02 to 6 wt% based on the aerosolisable formulation. In one aspect the acid is present in an amount of from 0.05 to 6 wt% based on the aerosolisable formulation. In one aspect the acid is present in an amount of from 0.08 to 6 wt% based on the aerosolisable formulation. In one aspect the acid is present in an amount of from 0.01 to 5 wt% based on the aerosolisable formulation. In one aspect the acid is present in an amount of from 0.02 to 5 wt% based on the aerosolisable formulation. In one aspect the acid is present in an amount of from 0.05 to 5 wt% based on the aerosolisable formulation. In one aspect the acid is present in an amount of from 0.08 to 5 wt% based on the aerosolisable formulation. In one aspect the acid is present in an amount of no greater than 4 wt% based on the aerosolisable formulation. In one aspect the acid is present in an amount of from 0.01 to 4 wt% based on the aerosolisable formulation. In one aspect the acid is present in an amount of from 0.02 to 4 wt% based on the aerosolisable formulation. In one aspect the acid is present in an amount of from 0.05 to 4 wt% based on the aerosolisable formulation. In one aspect the acid is present in an amount of from 0.08 to 4 wt% based on the aerosolisable formulation. In one aspect the acid is present in an amount of no greater than 3 wt% based on the aerosolisable formulation. In one aspect the acid is present in an amount of from 0.01 to 3 wt% based on the aerosolisable formulation. In one aspect the acid is present in an amount of from 0.02 to 3 wt% based on the aerosolisable formulation. In one aspect the acid is present in an amount of from 0.05 to 3 wt% based on the aerosolisable formulation. In one aspect the acid is present in an amount of from 0.08 to 3 wt% based on the aerosolisable formulation. In one aspect the acid is present in an amount of no greater than 2 wt% based on the aerosolisable formulation. In one aspect the acid is present in an amount of from 0.01 to 2 wt% based on the aerosolisable formulation. In one aspect the acid is present in an amount of from 0.02 to 2 wt% based on the aerosolisable formulation. In one aspect the acid is present in an amount of from 0.05 to 2 wt% based on the aerosolisable formulation. In one aspect the acid is present in an amount of from 0.08 to 2 wt% based on the aerosolisable formulation. In one aspect the acid is present in an amount of no greater than 1 wt% based on the aerosolisable formulation. In one aspect the acid is present in an amount of from 0.01 to 1 wt% based on the aerosolisable formulation. In one aspect the acid is present in an amount of from 0.02 to 1 wt% based on the aerosolisable formulation. In one aspect the acid is present in an amount of from 0.05 to 1 wt% based on the aerosolisable formulation. In one aspect the acid is present in an amount of from 0.08 to 1 wt% based on the aerosolisable formulation. In one aspect the acid is present in an amount of from 0.1 to 1 wt% based on the aerosolisable formulation. In one aspect the acid is present in an amount of no greater than 0.6 wt% based on the aerosolisable formulation. In one aspect the acid is present in an amount of from 0.01 to 0.6 wt% based on the aerosolisable formulation. In one aspect the acid is present in an amount of from 0.02 to 0.6 wt% based on the

aerosolisable formulation. In one aspect the acid is present in an amount of from 0.05 to 0.6 wt% based on the aerosolisable formulation. In one aspect the acid is present in an amount of from 0.08 to 0.6 wt% based on the aerosolisable formulation. In one aspect the acid is present in an amount of from 0.1 to 0.6 wt% based on the aerosolisable formulation. In one aspect the acid is present in an amount of no greater than 0.5 wt% based on the aerosolisable formulation. In one aspect the acid is present in an amount of from 0.01 to 0.5 wt% based on the aerosolisable formulation. In one aspect the acid is present in an amount of from 0.02 to 0.5 wt% based on the aerosolisable formulation. In one aspect the acid is present in an amount of from 0.05 to 0.5 wt% based on the aerosolisable formulation. In one aspect the acid is present in an amount of from 0.08 to 0.5 wt% based on the aerosolisable formulation. In one aspect the acid is present in an amount of no greater than 0.2 wt% based on the aerosolisable formulation. In one aspect the acid is present in an amount of from 0.01 to 0.2 wt% based on the aerosolisable formulation. In one aspect the acid is present in an amount of from 0.02 to 0.2 wt% based on the aerosolisable formulation. In one aspect the acid is present in an amount of from 0.05 to 0.2 wt% based on the aerosolisable formulation. In one aspect the acid is present in an amount of from 0.08 to 0.2 wt% based on the aerosolisable formulation. In one aspect the acid is present in an amount of no greater than 0.1 wt% based on the aerosolisable formulation. In one aspect the acid is present in an amount of from 0.01 to 0.1 wt% based on the aerosolisable formulation. In one aspect the acid is present in an amount of from 0.02 to 0.1 wt% based on the aerosolisable formulation. In one aspect the acid is present in an amount of from 0.05 to 0.1 wt% based on the aerosolisable formulation. In one aspect the acid is present in an amount of from 0.08 to 0.1 wt% based on the aerosolisable formulation.

The amount of acid and the solubility of the acid may be selected such that a given amount of the acid will dissolve in the water. In one aspect at 20 °C at least 20% of the acid dissolves in the water. In one aspect at 25 °C at least 20% of the acid dissolves in the water. In one aspect at 30 °C at least 20% of the acid dissolves in the water. In one aspect at 20 °C at least 35% of the acid dissolves in the water. In one aspect at 20 °C at least 40% of the acid dissolves in the water. In one aspect at 20 °C at least 45% of the acid dissolves in the water. In one aspect at 20 °C at least 50% of the acid dissolves in the water. In one aspect at 20 °C at least 55% of the acid dissolves in the water.

*Flavour*

The aerosolisable formulation comprises one or more flavours or flavouring components. As used herein, the terms "flavour" and "flavourant" refer to materials which, where local regulations permit, may be used to create a desired taste or aroma in a product for adult consumers. They may include extracts (e.g. liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

The one or more flavours may be selected from dodecan-1-ol; octan-1-ol; 4-methyl-1-propan-2-yl-7-oxabicyclo [2.2.1]heptane; 2,4,5-trimethylphenol; 2,4,6-trimethylphenol; 2,4-dimethylbenzaldehyde; 2-ethoxy-3-methylpyrazine; 2-ethylphenol; 2-ethylpyrazine; 2-methoxy-3-(2-methylpropyl)pyrazine; 5-methyl-2-propan-2-ylhex-2-enal; 2-methylpent-2-enoic acid; 2-butan-2-yl-3-methoxypyrazine; 3,5,5-trimethylcyclohexane-1,2-dione; 3-ethylphenol; [(z)-hex-3-enyl] 3-methylbutanoate; 3-methylnonane-2,4-dione; 3-methylbut-2-ene-1-thiol; 3-methyl-1h-indole; 3-methylphenol; 3-propylphenol; oxolan-2-one; 4-(hydroxymethyl)-2-methoxyphenol; 4-methylphenol; 2-methoxy-4-propylphenol; 4-propylphenol; [4-(3-oxobutyl)phenyl] acetate; 5-ethyl-2-methoxyphenol; (e)-5-methylhept-2-en-4-one; (e)-5-methyl-2-phenylhex-2-enal; 5-vinyl-2,3-dimethylpyrazine; 2-ethyl-6-methoxyphenol; 2-methoxy-6-methylphenol; 6-methylchromen-2-one; 1,1-diethoxyethane; 1-(4-methoxyphenyl)ethanone; acetic acid; 3-hydroxybutan-2-one; 1-phenylethanone; hexane-2,3-dione; 1-pyrazin-2-ylethanone; 1-(2-pyridyl)ethanone; 1-pyridin-4-ylethanone; 1-(1,3-thiazol-2-yl)ethanone; 1-(5-methylfuran-2-yl)ethanone; prop-2-enyl 6-cyclohexylhexanoate; prop-2-enyl hexanoate; prop-2-enyl nonanoate; pentyl butanoate; (2z)-2-(phenylmethylidene)heptanal; pentyl hexanoate; 1-methoxy-4-[(e)-prop-1-enyl]benzene; (4-methoxyphenyl)methyl acetate; (4-methoxyphenyl)methyl formate; benzaldehyde; dimethoxymethylbenzene; 4-methyl-2-phenyl-1,3-dioxolane; phenylmethyl acetate; phenylmethanol; 2-phenylethanol; phenylmethyl 3-phenylprop-2-enoate; phenylmethyl formate; phenylmethyl 2-phenylacetate; 1,7,7-trimethylbicyclo[2.2.1]heptan-6-ol; (1-methyl-2-oxo-propyl) butanoate; 2,3-dihydroxybutane; butan-1-ol; butyl 2-methylbutanoate; butyl acetate; butyl butanoate; (1-butoxy-1-oxopropan-2-yl) butanoate; butyl 3-methylbutanoate; 5-butyl-4-methyloxolan-2-one; butanoic acid; oxolan-2-one; 2-methyl-5-propan-2-ylphenol; 2-methyl-5-prop-1-en-2-ylcyclohex-2-en-1-ol; 4-methyl-1-propan-2-ylcyclohex-3-en-1-ol; 2-methyl-5-prop-1-en-2-ylcyclohex-2-en-1-one; (5s)-2-methyl-5-prop-1-en-2-ylcyclohex-2-en-1-one;

(5r)-2-methyl-5-prop-1-en-2-ylcyclohex-2-en-1-one; (1r,4e,9s)-4,11,11-trimethyl-8-methylidenebicyclo[7.2.0]undec-4-ene; 4,5-epoxy-4,11,11-trimethyl-8-methylenebicyclo(7.2.0)undecane; (e)-3-phenylprop-2-enal; 3-phenylprop-2-enoic acid; [(e)-3-phenylprop-2-enyl] acetate; [(e)-3-phenylprop-2-enyl] (e)-3-phenylprop-2-enoate; z-dec-4-enal; (z)-non-6-en-1-ol; 3,7-dimethylocta-2,6-dienal; 3,7-dimethyloct-6-enal; 3,7-dimethyloct-6-en-1-ol; 3,7-dimethyloct-6-enyl acetate; (e)-1-(2,6,6-trimethyl-1-cyclohexa-1,3-dienyl)but-2-en-1-one; (e)-1-(2,6,6-trimethyl-1-cyclohex-2-enyl)but-2-en-1-one; 1-(2,6,6-trimethyl-1-cyclohexenyl)but-2-en-1-one; 6-pentyloxan-2-one; 5-hexyloxolan-2-one; decanal; decanoic acid; diethyl propanedioate; 2,3-diethylpyrazine; diethyl decanedioate; chroman-2-one; 3-methyl-2-pentylcyclopent-2-en-1-one; 1,3-dimethoxybenzene; 1,4-dimethoxybenzene; 2,6-dimethoxyphenol; (2-methyl-1-phenylpropan-2-yl) butanoate; 2,3-dimethylpyrazine; 2,5-dimethylpyrazine; 2,6-dimethylpyrazine; methylsulfanyldisulfanylmethane; 3,4-dimethylcyclopentane-1,2-dione; 3-hydroxy-4,5-dimethyl-5h-furan-2-one; 2,6-dimethylhept-5-enal; 4-hydroxy-2,5-dimethylfuran-3-one; 2,6-dimethylpyridine; phenoxybenzene; 6-heptyloxan-2-one; 5-octyloxolan-2-one; ethyl 3-methylsulfanylpropanoate; ethyl 3-hydroxybutanoate; ethyl acetate; ethyl 3-oxobutanoate; ethyl benzoate; ethyl butanoate; ethyl 3-phenylprop-2-enoate; ethyl decanoate; ethyl formate; 4-ethyl-2-methoxyphenol; ethyl heptanoate; ethyl hexanoate; ethyl 2-methylpropanoate; ethyl 3-methylbutanoate; ethyl 2-hydroxypropanoate; ethyl dodecanoate; ethyl 4-oxopentanoate; 2-ethyl-3-hydroxypyran-4-one; ethyl 3-methyl-3-phenyloxirane-2-carboxylate; ethyl tetradecanoate; ethyl nonanoate; ethyl octanoate; ethyl (z)-octadec-9-enoate; ethyl hexadecanoate; ethyl propanoate; ethyl (e)-but-2-enoate; ethyl (e)-oct-2-enoate; ethyl pentanoate; 3-ethoxy-4-hydroxybenzaldehyde; 2-ethyl-3,5-dimethylpyrazine; 3-ethyl-2-hydroxycyclopent-2-en-1-one; ethyl 2-methylbutanoate; ethyl 2-methylpentanoate; 2-ethyl-3-methylpyrazine; 2-ethyl-3,5-dimethylpyrazine; ethyl-3-hexenoate; 5-ethyl-3-hydroxy-4-methyl-5h-furan-2-one; 5-ethyl-4-hydroxy-2-methylfuran-3-one; 4-ethylphenol; 3-ethylpyridine; 4-ethylpyridine; 4,7,7-trimethyl-8-oxabicyclo[2.2.2]octane; 2-methoxy-4-prop-2-enylphenol; (1s,4r,6s)-1,5,5-trimethylbicyclo[2.2.1]heptan-6-ol; furan-2-ylmethyl acetate; furan-2-ylmethanethiol; furan-2-ylmethyl propanoate; (2e)-3,7-dimethylocta-2,6-dien-1-ol; [(2e)-3,7-dimethylocta-2,6-dienyl] acetate; (5e)-6,10-dimethylundeca-5,9-dien-2-one; [(2e)-3,7-dimethylocta-2,6-dienyl] formate; (2r,3s,4r,5r)-2,3,4,5,6-pentahydroxyhexanoic acid; 1,3-diacetyloxypropan-2-yl acetate; 2-methoxyphenol; 5-propyloxolan-2-one; heptanoic acid; heptan-2-one; heptan-1-ol; oxacycloheptadec-7-en-2-one; 6-methyloxan-2-one; 5-ethyloxolan-2-one; hexanal; hexanoic acid; hexan-1-ol; (z)-hex-3-en-1-ol; [(z)-hex-3-enyl] acetate; hex-2-enal; hex-3-enoic acid; (e)-hex-2-enoic acid; hex-2-en-1-ol; hex-2-enyl acetate; [(z)-hex-3-enyl] butanoate; [(z)-hex-3-enyl] formate; hex-3-enyl 2-methylbutanoate; hexyl 2-methylbutanoate; hexyl acetate; hexyl butanoate; hexyl formate; hexyl hexanoate; hexyl 2-hydroxypropanoate; hexyl octanoate; (1e,4e,8e)-2,6,6,9-tetramethylcycloundeca-1,4,8-triene; 2-hydroxy-4-methylbenzaldehyde; 4-hydroxy-5-methylfuran-3-one; 7-hydroxy-3,7-dimethyloctanal; 4-(4-hydroxyphenyl)butan-2-one; 4-(2,6,6-trimethyl-1-cyclohex-2-enyl) but-3-en-2-one; 4-(2,6,6-trimethyl-1-cyclohexenyl)but-3-en-2-one; (e)-4-[(1s,5r)-2,5,6,6-tetramethyl-1-cyclohex-2-enyl] but-3-en-2-one; 3-methylbutyl acetate; 3-methylbutyl butanoate; 3-methylbutyl hexanoate; 3-methylbutyl 3-methylbutanoate; 3-methylbutyl 2-hydroxypropanoate; 3-methylbutyl octanoate; 3-methylbutyl propanoate; 3-methylbutyl pentanoate; [(1s,4r,6s)-1,7,7-trimethyl-6-bicyclo[2.2.1]heptanyl] acetate; 2-methylpropan-1-ol; 2-methylpropyl acetate; 3-oxo-butanoic acid, 2-methylpropyl ester; 2-methylpropyl butanoate; 2-methylpropyl hexanoate; 2-methylpropyl 3-methylbutanoate; 2-methylpropyl 2-methylbutanoate; 2-methylpropanal; 2-methylpropanoic acid; 1,2-dimethoxy-4-prop-1-enylbenzene; 5-methyl-2-propan-2-ylcyclohexan-1-one; propan-2-yl tetradecanoate; 5-methyl-2-prop-1-en-2-ylcyclohexan-1-ol; 3-methylbutanoic acid; 3-methyl-2-[(z)-pent-2-enyl]cyclopent-2-en-1-one; 2,6,6-trimethylcyclohex-2-ene-1,4-dione; dodecanal; (4r)-1-methyl-4-prop-1-en-2-ylcyclohexene; 3,7-dimethylocta-1,6-dien-3-ol; 2-(5-methyl-5-vinyltetrahydro-2-furanyl)-2-propanol; 3,7-dimethylocta-1,6-dien-3-yl acetate; 3,7-dimethylocta-1,6-dien-3-yl butanoate; 3-hydroxy-2-methylpyran-4-one; (4z)-4-[(e)-but-2-enylidene]-3,5,5-trimethylcyclohex-2-en-1-one; 1-methyl-4-propan-2-ylcyclohexa-1,4-diene; 5-methyl-2-(2-sulfanylpropan-2-yl)cyclohexan-1-one; (1r,2s,5r)-5-methyl-2-propan-2-ylcyclohexan-1-ol; (2s,5r)-2-isopropyl-5-methylcyclohexanone; [(6s,9r)-9-methyl-6-propan-2-yl-1,4-dioxaspiro[4.5]decan-3-yl]methanol; (5-methyl-2-propan-2-ylcyclohexyl) acetate; (5-methyl-2-propan-2-ylcyclohexyl) 2-methylbutanoate; 3-methylsulfanylpropanal; 4-methoxybenzaldehyde; 2-methoxy-3-methyl-pyrazine; 2-methoxy-4-methylphenol; 4-ethenyl-2-methoxyphenol; p-anisyl alcohol; 4-(4-methoxyphenyl)butan-2-one; 2-methyl -2-pentenoic acid; 1-(4-methylphenyl)ethanone; 1-methoxy-4-methylbenzene; methyl 2-aminobenzoate; 1-phenylethyl acetate; 2-methylbutan-1-ol; 2-methylbutyl acetate; 3-methylbutyl 2-methylpropanoate; 2-methylbutanal; 3-methylbutanal; methyl butanoate; 2-methylbutanoic acid; methyl (e)-3-phenylprop-2-enoate; 3-methylcyclopentane-1,2-dione; methyl 2-(3-oxo-2-pentylcyclopentyl)acetate; 5-methylfuran-2-carbaldehyde; 2-(methyldisulfanylmethyl)furan; methyl hexanoate; methyl 2-methylpropanoate; methyl 2-methylaminobenzoate; methyl 4-methoxybenzoate; 3-methylpentanoic acid; methyl 2-phenylacetate; 2-methylpyrazine; 5-methylquinoxaline; methylsulfanylmethane; 2-methyloxolan-3-one; s-methyl butanethioate; methyl (e)-non-2-enoate; 2-methylpentanoic acid; 3-methylcyclohexane-1,2-dione; methyl furan-2-carboxylate; methyl 2-methylbutanoate; 1-(1h-pyrrol-2-yl)ethanone; methyl 3-methylsulfanylpropanoate; 6-methylhepta-3,5-dien-2-one; 6-methylhept-5-en-2-one; 2-(4-methyl-1,3-thiazol-5-yl)ethanol; 5-methyl-6,7-dihydro-5h-cyclopenta[b]pyrazine; (e)-1-(2,6,6-trimethyl-1-cyclohex-2-enyl)pent-1-en-3-one; 3-methylbutan-1-ol; 3-methylpyridine; 4-methylpyridine; 6-methylquinoline; 5-methylthiophene-2-carbaldehyde; (e)-2-methylbut-2-enoic acid; (1s,2s,5r)-5-methyl-2-propan-2-ylcyclohexan-1-ol; (2z)-3,7-dimethylocta-2,6-dien-1-ol; 3,7,11-trimethyldodeca-1,6,10-trien-3-ol; [(2z)-3,7-dimethylocta-2,6-dienyl] acetate;

(2e,6z)-nona-2,6-dienal; 2,6-nonadien-1-ol; 6-butyloxan-2-one; 5-pentyltetrahydrofuran-2-one; nonanal; nonanoic acid; nonan-2-one; (z)-non-6-enal; (3e)-3,7-dimethylocta-1,3,6-triene; 6-propyloxan-2-one; 5-butyloxolan-2-one; octanal; octanoic acid; oct-1-en-3-ol; octyl acetate; (e)-octadec-9-enoic acid; 5-methyl-3h-furan-2-one; 1-oxacyclohexadecan-2-one; pentan-1-ol; pentan-2-one; 2-phenylethyl 3-methylbutanoate; 2-phenylethyl 2-phenylacetate; 2-phenylacetaldehyde; 2-phenylacetic acid; 3-phenylpropanoic acid; 3-phenylpropan-1-ol; 2-phenyl-2-butenal; 3-phenyl-2-propen-1-ol; 4,7,7-trimethylbicyclo[3.1.1]hept-3-ene; 7,7-dimethyl-4-methylidenebicyclo[3.1.1]heptane; (6s)-3-methyl-6-propan-2-ylcyclohex-2-en-1-one; 1,3-benzodioxole-5-carbaldehyde; 2-ethoxy-5-[(e)-prop-1-enyl]phenol; propanoic acid; propyl acetate; propyl butanoate; propyl formate; 3-propylidene-2-benzofuran-1-one; 2-oxopropanoic acid; 3,7-dimethyloct-6-en-1-ol; (2r,3r)-2,3-dihydroxysuccinic acid; 2-[(1s)-4-methyl-1-cyclohex-3-enyl]propan-2-ol; 1-methyl-4-propan-2-ylidenecyclohexene; 2-(4-methyl-1-cyclohex-3-enyl)propan-2-yl acetate; 5,6,7,8-tetrahydroquinoxaline; 2,3,5,6-tetramethylpyrazine; 2,6,6,10-tetramethyl-1-oxaspiro[4.5]dec-9-ene; 5-methyl-2-propan-2-ylphenol; (4-methylphenyl) acetate; 4-methylbenzaldehyde; (4-methylphenyl) 3-methylbutanoate; e-2-methoxy-4-prop-1-enylphenol; 2,3,5-trimethylphenol; 2,3,5-trimethylpyrazine; 6-hexyloxan-2-one; 5-heptyloxolan-2-one; undecan-2-one; pentanal; pentanoic acid; 5-methyloxolan-2-one; 4-hydroxy-3-methoxybenzaldehyde; 3,4-dimethoxybenzaldehyde; 2-phenylethyl acetate; (e)-hex-2-enoic acid; (3ar,5as,9as,9br)-3a,6,6,9a-tetramethyldodecahydronaphtho[2,1-b]furan; 6-pentyl-5,6-dihydropyran-2-one; 2-phenylethyl 2-methylpropanoate; 2-methyl-1[1-(2-methylbutoxy)ethoxy]butane; 4,4a-dimethyl-6-prop-1-en-2-yl-3,4,5,6,7,8-hexahydronaphthalen-2-one; 2-(1-Mercapto-1-methylethyl)-5-methylcyclohexanone; and mixtures thereof

The one or more flavours may be selected from 4-(4-Methoxyphenyl)-2-butanone [also known as (4-(para-) methoxyphenyl)-2-butanone], 4-Hydroxy-3-methoxybenzaldehyde [also known as vanillin], 5-heptyloxolan-2-one [also known as γ-undecalactone], (2S,5R)-2-Isopropyl-5-methylcyclohexanone [also known as menthone], 2-ethoxy-5-[(E)-prop-1-enyl]phenol [also known as 5-propenyl guaethol], (1R,2S,5R)-5-methyl-2-propan-2-ylcyclohexan-1-ol [also known as menthol], 2-(1-Mercapto-1-methylethyl)-5-methylcyclohexanone [also known as para-mentha-8-thiol-3-one] and mixtures thereof. In one aspect the flavour is at least menthol.

If present, the one or more flavours may be present in any suitable amount. In one aspect the one or more flavours are present in a total amount of no greater than 10 wt.% based on the aerosolisable formulation. In one aspect the one or more flavours are present in a total amount of no greater than 7 wt.% based on the aerosolisable formulation. In one aspect the one or more flavours are present in a total amount of no greater than 5 wt.% based on the aerosolisable formulation. In one aspect the one or more flavours are present in a total amount of no greater than 4 wt.% based on the aerosolisable formulation. In one aspect the one or more flavours are present in a total amount of no greater than 3 wt.% based on the aerosolisable formulation. In one aspect the one or more flavours are present in a total amount of no greater than 2wt.% based on the aerosolisable formulation. In one aspect the one or more flavours are present in a total amount of no greater than 1wt.% based on the aerosolisable formulation.

In one aspect the one or more flavours are present in a total amount of from 0.01 to 5wt.% based on the aerosolisable formulation. In one aspect the one or more flavours are present in a total amount of from 0.01 to 4wt.% based on the aerosolisable formulation. In one aspect the one or more flavours are present in a total amount of from 0.01 to 3wt.% based on the aerosolisable formulation. In one aspect the one or more flavours are present in a total amount of from 0.01 to 2wt.% based on the aerosolisable formulation. In one aspect the one or more flavours are present in a total amount of from 0.01 to 1wt.% based on the aerosolisable formulation. In one aspect the one or more flavours are present in a total amount of from 0.01 to 0.5wt.% based on the aerosolisable formulation.

*Encapsulating Material*

The aerosolisable formulation comprises one or more encapsulating materials. The one or more encapsulating materials may be present in any suitable amount in the aerosolisable formulation. In one aspect the one or more encapsulating materials are present in a total amount of no greater than 12 wt.% based on the aerosolisable formulation. In one aspect the one or more encapsulating materials are present in a total amount of no greater than 10 wt.% based on the aerosolisable formulation. In one aspect the one or more encapsulating materials are present in a total amount of no greater than 9 wt.% based on the aerosolisable formulation. In one aspect the one or more encapsulating materials are present in a total amount of no greater than 8 wt.% based on the aerosolisable formulation. In one aspect the one or more encapsulating materials are present in a total amount of no greater than 7 wt.% based on the aerosolisable formulation. In one aspect the one or more encapsulating materials are present in a total amount of no greater than 6 wt.% based on the aerosolisable formulation. In one aspect the one or more encapsulating materials are present in a total amount of no greater than 5 wt.% based on the aerosolisable formulation. In one aspect the one or more encapsulating materials are present in a total amount of no greater than 4 wt.% based on the aerosolisable formulation. In one aspect the one or more encapsulating materials are present in a total amount of no greater than 3 wt.% based on the aerosolisable formulation. In one aspect the one or more encapsulating materials are present in a total amount of no greater than 2 wt.% based on the aerosolisable formulation. In one aspect the one or more encapsulating materials are present in a total amount of no

greater than 1 wt.% based on the aerosolisable formulation. In one aspect the one or more encapsulating materials are present in a total amount of no greater than 0.1 wt.% based on the aerosolisable formulation. In one aspect the one or more encapsulating materials are present in a total amount of no greater than 0.01 wt.% based on the aerosolisable formulation. In one aspect the one or more encapsulating materials are present in a total amount of no greater than 0.001 wt.% based on the aerosolisable formulation.

The one or more cyclodextrins are selected from the group consisting of substituted (α)-cyclodextrin, substituted (β)-cyclodextrin, substituted (γ)-cyclodextrin, and mixtures thereof.

In one aspect the one or more cyclodextrins is selected from substituted (β)-cyclodextrins. Chemical substitutions at the 2-, 3-, and 6-hydroxyl sites are envisaged, and in particular substitution at the 2-position.

In one aspect the one or more cyclodextrins are selected from the group consisting of 2-hydroxy-propyl-α-cyclodextrin, 2-hydroxy-propyl-β-cyclodextrin, 2-hydroxy-propyl-γ-cyclodextrin and mixtures thereof. In one aspect the one or more cyclodextrins is at least 2-hydroxy-propyl-α-cyclodextrin. In one aspect the one or more cyclodextrins is at least 2-hydroxy-propyl-β-cyclodextrin. In one aspect the one or more cyclodextrins is at least 2-hydroxy-propyl-γ-cyclodextrin.

2-hydroxy-propyl derivatives of cyclodextrins, such as 2-hydroxy-propyl-β-cyclodextrin have increased solubility in water when compared to base cyclodextrins such as β-cyclodextrin.

One or more cyclodextrins may or may not be present in any suitable amount in the aerosolisable formulation. In one aspect the one or more cyclodextrins are present in a total amount of no greater than 12 wt.% based on the aerosolisable formulation. In one aspect the one or more cyclodextrins are present in a total amount of no greater than 10 wt.% based on the aerosolisable formulation. In one aspect the one or more cyclodextrins are present in a total amount of no greater than 9 wt.% based on the aerosolisable formulation. In one aspect the one or more cyclodextrins are present in a total amount of no greater than 8 wt.% based on the aerosolisable formulation. In one aspect the one or more cyclodextrins are present in a total amount of no greater than 7 wt.% based on the aerosolisable formulation. In one aspect the one or more cyclodextrins are present in a total amount of no greater than 6 wt.% based on the aerosolisable formulation. In one aspect the one or more cyclodextrins are present in a total amount of no greater than 5 wt.% based on the aerosolisable formulation. In one aspect the one or more cyclodextrins are present in a total amount of no greater than 4 wt.% based on the aerosolisable formulation. In one aspect the one or more cyclodextrins are present in a total amount of no greater than 3 wt.% based on the aerosolisable formulation. In one aspect the one or more cyclodextrins are present in a total amount of no greater than 2 wt.% based on the aerosolisable formulation. In one aspect the one or more cyclodextrins are present in a total amount of no greater than 1 wt.% based on the aerosolisable formulation. In one aspect the one or more cyclodextrins are present in a total amount of no greater than 0.1 wt.% based on the aerosolisable formulation. In one aspect the one or more cyclodextrins are present in a total amount of no greater than 0.01 wt.% based on the aerosolisable formulation. In one aspect the one or more cyclodextrins are present in a total amount of no greater than 0.001 wt.% based on the aerosolisable formulation.

As discussed herein, in one aspect the present invention provides an aerosolisable formulation comprising (i) water (ii) one or more flavours to be encapsulated; and (iii) one or more encapsulating materials selected from the group consisting of substituted (α)-cyclodextrin, substituted (β)-cyclodextrin, substituted (γ)-cyclodextrin, and mixtures thereof having a solubility in water of at least 50% of the solubility in water of the one or more flavours to be encapsulated; and (iv) nicotine.

In one aspect, the one or more encapsulating materials have a solubility in water of at least 55 % of the solubility in water of the one or more flavours to be encapsulated. In one aspect, the one or more encapsulating materials have a solubility in water of at least 60 % of the solubility in water of the one or more flavours to be encapsulated. In one aspect, the one or more encapsulating materials have a solubility in water of at least 65 % of the solubility in water of the one or more flavours to be encapsulated. In one aspect, the one or more encapsulating materials have a solubility in water of at least 70 % of the solubility in water of the one or more flavours to be encapsulated. In one aspect, the one or more encapsulating materials have a solubility in water of at least 75 % of the solubility in water of the one or more flavours to be encapsulated. In one aspect, the one or more encapsulating materials have a solubility in water of at least 80 % of the solubility in water of the one or more flavours to be encapsulated. In one aspect, the one or more encapsulating materials have a solubility in water of at least 85 % of the solubility in water of the one or more flavours to be encapsulated. In one aspect, the one or more encapsulating materials have a solubility in water of at least 90 % of the solubility in water of the one or more flavours to be encapsulated. In one aspect, the one or more encapsulating materials have a solubility in water of at least 95 % of the solubility in water of the one or more flavours to be encapsulated.

*Encapsulating Material and Flavour*

The one or more encapsulating materials and the flavour may be present in any suitable amount relative to each other. The molar ratio of encapsulating material to flavour may be selected as desired. In one aspect the molar ratio of encapsulating material to flavour is from 5:1 to 1:5. In one aspect the molar ratio of encapsulating material to flavour is from

4:1 to 1:4. In one aspect the molar ratio of encapsulating material to flavour is from 3:1 to 1:3. In one aspect the molar ratio of encapsulating material to flavour is from 2:1 to 1:2. In one aspect the molar ratio of encapsulating material to flavour is from 1.5:1 to 1:1.5. In one aspect the molar ratio of encapsulating material to flavour is from 1.2:1 to 1:1.2. In one aspect the molar ratio of encapsulating material to flavour is from 5:1 to 1:1. In one aspect the molar ratio of encapsulating material to flavour is from 4:1 to 1:1. In one aspect the molar ratio of encapsulating material to flavour is from 3:1 to 1:1. In one aspect the molar ratio of encapsulating material to flavour is from 2:1 to 1:1. In one aspect the molar ratio of encapsulating material to flavour is from 1.5:1 to 1:1. In one aspect the molar ratio of encapsulating material to flavour is from 1.4:1 to 1:1. In one aspect the molar ratio of encapsulating material to flavour is from 1.3:1 to 1:1. In one aspect the molar ratio of encapsulating material to flavour is from 1.2:1 to 1:1. In one aspect the molar ratio of encapsulating material to flavour is from 1.1:1 to 1:1. In one aspect the molar ratio of encapsulating material to flavour is approximately 1:1.

*Process*

As discussed herein, in one aspect there is provided a process for improving the sensory properties of an aerosolised nicotine formulation, the process comprising the steps of aerosolising an aerosolisable formulation comprising

(i) water;
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

As discussed herein, in one aspect there is provided a process for forming an aerosol, the process comprising aerosolising an aerosolisable formulation comprising (i) water (ii) one or more flavours to be encapsulated; and (iii) one or more encapsulating materials selected from the group consisting of substituted ($\alpha$)-cyclodextrin, substituted ($\beta$)-cyclodextrin, substituted ($\gamma$)-cyclodextrin, and mixtures thereof; wherein in the presence of water the energy of binding of the one or more encapsulating materials with the one or more flavours to be encapsulated is from -0.5 to -8 kcal/mol.

In the process the aerosol may be formed by a process performed at a temperature below 60°C.In the process the aerosol may be formed by a process performed at a temperature below 50°C. In the process the aerosol may be formed by a process performed at a temperature below 40°C. In the process the aerosol may be formed by a process performed at a temperature below 30°C. In the process the aerosol may be formed by a process performed at a temperature below 25°C. In the process the aerosol may be formed by a process which does not involve heating.

In the process the aerosol may be formed by applying ultrasonic energy to the aerosolisable formulation.

In one aspect the aerosol the aerosol of the aerosolised formulation has a D50 of from 2 to 6$\mu$m. References in the present specification to particle size distribution, D50, D10 or D90 refer to values measured in accordance with British and European Pharmacopoeia, 2.9.31 Particle Size Analysis By Laser Light Diffraction (see BRITISH PHARMACOPOEIA COMMISSION. (2014), British Pharmacopoeia. London, England: Stationery Office and COUNCIL OF EUROPE. (2013). European Pharmacopoeia. Strasbourg, France: Council of Europe). The terms D50, Dv50 and Dx50 are interchangeable. The terms D10, Dv10 and Dx10 are interchangeable. The terms D90, Dv90 and Dx90 are interchangeable.

In one aspect the aerosol has a D50 of from 2.5 to 6$\mu$m. In one aspect the aerosol has a D50 of from 3 to 6$\mu$m. In one aspect the aerosol has a D50 of from 3.5 to 6$\mu$m. In one aspect the aerosol has a D50 of from 4 to 6$\mu$m. In one aspect the aerosol has a D50 of from 4.5 to 6$\mu$m. In one aspect the aerosol has a D50 of from 5 to 6$\mu$m. In one aspect the aerosol has a D50 of from 2.5 to 5.5$\mu$m. In one aspect the aerosol has a D50 of from 3 to 5.5$\mu$m. In one aspect the aerosol has a D50 of from 3.5 to 5.5$\mu$m. In one aspect the aerosol has a D50 of from 4 to 5.5$\mu$m. In one aspect the aerosol has a D50 of from 4.5 to 5.5$\mu$m. In one aspect the aerosol has a D50 of from 5 to 5.5$\mu$m.

In one aspect the aerosol has a D10 of at least 0.5$\mu$m. In one aspect the aerosol has a D10 of at least 1$\mu$m. In one aspect the aerosol has a D10 of at least 2$\mu$m.

In one aspect the aerosol has a D90 of no greater than 15$\mu$m. In one aspect the aerosol has a D90 of no greater than 12$\mu$m. In one aspect the aerosol has a D90 of no greater than 10$\mu$m.

In one aspect D50 is measured after exclusion of particles having a particle size of less than 1$\mu$m. In one aspect D10 is measured after exclusion of particles having a particle size of less than 1$\mu$m. In one aspect D90 is measured after exclusion of particles having a particle size of less than 1$\mu$m.

The formulation may be contained or delivered by any means. In one aspect the present invention provides a contained aerosolisable formulation comprising (a) one or more containers; and (b) an aerosolisable formulation as defined herein. The container may be any suitable container, for example to allow for the storage or delivery of the formulation. In one aspect the container is configured for engagement with an electronic aerosol provision system. The

container may be configured to become fluidly in communication with an electronic aerosol provision system so that formulation may be delivered to the electronic aerosol provision system. As described above, the present disclosure relates to container which may be used in an electronic aerosol provision system, such as an e-cigarette. Throughout the following description the term "e-cigarette" is used; however, this term may be used interchangeably with electronic aerosol provision system.

As discussed herein, the container of the present invention is typically provided for the delivery of aerosolisable formulation to or within an e-cigarette. The aerosolisable formulation may be held within an e-cigarette or may be sold as a separate container for subsequent use with or in an e-cigarette. As understood by one skilled in the art, e-cigarettes may contain a unit known as a detachable cartomiser which typically comprises a reservoir of aerosolisable formulation, an aerosoliser such as a wick material and a heating element for vaporising the aerosolisable formulation. In some e-cigarettes, the cartomiser is part of a single-piece device and is not detachable. In one aspect the container is a cartomiser or is part of a cartomiser. In one aspect the container is not a cartomiser or part of a cartomiser and is a container, such as a tank, which may be used to deliver nicotine formulation to or within an e-cigarette.

In one aspect the container is part of an e-cigarette. Therefore in a further aspect the present invention provides an electronic aerosol provision system comprising: an aerosolisable formulation as defined herein; an aerosoliser for aerosolising formulation for inhalation by a user of the electronic aerosol provision system; and a power supply comprising a cell or battery for supplying power to the aerosoliser.

In addition to the aerosolisable formulation of the present invention and to systems such as containers and electronic aerosol provision systems containing the same, the present invention provides a process for improving the sensory properties of an aerosolised nicotine.

Reference to an improvement in the sensory properties of a vaporised nicotine solution refer may include an improvement in the smoothness of the vaporised nicotine solution as perceived by a user.

The process of the present invention may comprises additional steps either before the steps listed, after the steps listed or between one or more of the steps listed.

**BRIEF DESCRIPTION OF THE DRAWINGS**

The present invention will now be described in further detail by way of example only with reference to the accompanying figure in which:-

Figure 1 shows a graph illustrating variation of $p_sK_{a2}$ with nicotine concentration; and
Figures 2 to 4 show docking schematics.

The invention will now be described with reference to the following non-limiting example.

**Example**

The binding and energy of binding of hydroxypropyl beta cyclodextrin and menthol was studied with automated docking simulations. Automated docking simulations use a three-dimensional representation to assess the fit for a guest substrate in a molecular cavity. It is generally accepted that the predicted binding energies may be accurately determined with automated docking simulations.

Docking was performed in Autodock 4.2 [The Scripps Research Institute, La Jolla, CA, USA] (Morris, et al., 2009) using settings as detailed below. In brief, hydrogens were merged and Kolman/Gasteiger charges were added according to standard methodology. Grid size was adjusted where necessary to accommodate the receptor. In general, settings were left at default values. The receptors were treated as rigid entities. Output format was Lamarckian genetic algorithm.

**Host - Hydroxypropyl Beta Cyclodextrin**

Hydroxypropyl-beta-CD (HP-β-CD) contains numerous isomers, due to random substitution during synthesis. The primary hydroxyl groups at the C-6 of the sugars are the most likely to be substituted owing to their nucleophilicity and lack of steric crowding, but substitution can also occur at the C-2 and C-3 positions, which are at the opposite face of the cavity to C-6. The crystal structures of beta (β) cyclodextrin were taken from The Cambridge Crystallographic Data Centre (CCDC) (item designation "ARUXIU") and modified with hydroxypropyl groups according to several substitution patterns to see how important this is to binding.

The following variants were trialled:

Version 1: No substitution of the beta cyclodextrin
Version 2: Five (out of seven) of the C-6 hydroxyls were functionalised, along with one C-2 and one C-3, all selected at

random
Version 3: Five of the C-6 hydroxyls were functionalised, selected at random

These modifications were made using Discovery Studio Visualizer [v16.1.0.15350, (2015), Dassault Systémes Biovar Corp]. The structures were then optimized with the fast, Dreiding-like force field tool within Discovery Studio Visualizer and converted to .pdb format for onward processing using Autodock 4.2. The settings used in the modelling each of Versions 1, 2 and 3 are provided in the table below.

| Setting | Further information | Simulation version | | |
|---|---|---|---|---|
| | | 1 | 2 | 3 |
| Grid Point Spacing | Angstroms | 0.375 | 0.375 | 0.375 |
| Number of grid points in each Cartesian direction | x | 40 | 40 | 50 |
| | y | 40 | 40 | 40 |
| | z | 40 | 40 | 50 |
| Coordinates of Central Grid Point of Map | | (4.422, 6.023, 14.126) | (4.793, 6.323, 14.339) | (4.445, 6.360, 14.413) |
| Minimum coordinates in grid | | (-3.078, -1.477, 6.626) | (-2.707, -1.177, 6.839) | (-4.930, -1.140, 5.038) |
| Maximum coordinates in grid | | (11.922, 13.523, 21.626) | (12.293, 13.823, 21.839) | (13.820, 13.860, 23.788) |
| User-specified initial position for ligand | | random | | |
| Initial relative dihedral offset | | random | | |
| User-specified initial relative dihedrals | | random | | |
| Docking search parameter | | Genetic Algorithm | | |
| Number of requested GA dockings | | 10 runs | | |
| Population size | | 150 | | |
| Maximum number of evaluations | | 2500000 | | |
| Maximum number of top individuals that automatically survive | | 1 | | |
| Rate of gene mutation | | 0.02 | | |
| Rate of crossover | | 0.08 | | |
| GA crossover mode | | "twopt" | | |
| Mean of Cauchy distribution for gene mutation | alpha parameter | 0 | | |
| Variance of Cauchy distribution for gene mutation | beta parameter | 1 | | |
| Number of generations fro picking worst individuals | | 10 | | |
| Docking output | | Lamarckian GA | | |

**Guest - Menthol**

The 3-dimensional structure for menthol was obtained from Pubchem and converted to .pdb format for use in docking simulation.

**Docking Results**

The docking simulation defaults to 10 repetitions to check for viable conformations between host and guest. Therefore, each simulation gives 10 results. The result is expressed in terms of Gibbs free energy of binding. A negative value denotes an energetically favoured process. The absolute size of this binding energy is a useful comparator of binding affinity. As a general rule, negative values with an absolute size above around 5 Kcal / mole indicate moderately strong binding affinity. The full data for each version are given in docking log files herein but are summarised below, along with an image showing a representative binding complex for each version.

Version 1: Unsubstituted beta cyclodextrin

The docking simulation gave several conformations, in all cases binding the guest within the cavity of the host - as typified by Figure 2, which shows atomic spheres for the host and a line representation for the guest to improve clarity. The corresponding docking log file is named menBCD.dlg.

The runs provided a free energy of binding of -5.1 Kcal/mole.

Version 2: Hydroxypropyl beta cyclodextrin substituted at 7 sites

The docking simulation gave several conformations, in all cases binding the guest within the cavity of the host - as typified by Figure 3, which shows atomic spheres for the host and a line representation for the guest to improve clarity.

The runs provided a free energy of binding of -6.0 Kcal/mole.

Version 3: Hydroxypropyl beta cyclodextrin substituted at 5 points

The docking simulation gave several conformations, in all cases binding the guest within the cavity of the host - as typified by Figure 4, which shows atomic spheres for the host and a line representation for the guest to improve clarity.

The runs provided a free energy of binding of -5.6 Kcal/mole.

Summary and Conclusions

The binding of menthol in hydroxypropyl beta cyclodextrin can be modelled using molecular docking, which establishes a binding free energy of between -5 and -6 Kcal/mole.

The extent and location of the hydroxypropyl groups in HP-β-CD can vary from molecule to molecule. We therefore checked the docking process using a range of possible structures to ensure sensitivity of the measurement towards fluctuations in structure. Two versions of the hydroxypropyl derivative of cyclodextrin were assessed, and gave broadly similar results. An unfunctionalized variant was also assessed, and gave reasonably similar results.

Various modifications and variations of the present invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.

Aspects of the invention are also provided in which Hansen sphere values for one or more encapsulating materials are selected to achieve desirable compatibility with flavours included in the aerosolisable formulation, and with nicotine, if present. As will be understood by one skilled in the art Hansen sphere values describe the interaction between flavours and the encapsulating material. The relevant measurements are:

δD - Measure of dispersion forces
δP - Measure of polar (dipolar) interactions
δH - Measure of hydrogen bonding

The closer these values are for flavour and solvent (water) the more soluble they will be. Through selection of the Hansen sphere values the encapsulating material encapsulates at least one of the one or more flavours in preference to other components such as nicotine.

As will be understood by one skilled in the art compatibility between encapsulating material (host) and the encapsulated species (guest) can be defined as how "alike" they are. This can also be measured using the Hansen Solubility Parameters (HSP) Distance (also termed Ra in the equation below) between the host and guest molecules.

$$Ra^2 = 4(\delta D_1 - \delta D_2)^2 + (\delta P_1 - \delta P_2)^2 + (\delta H_1 - \delta H_2)^2$$

where,

*Ra* = HSP Distance
$\delta D$ = The energy from dispersion forces between molecules
$\delta P$ = The energy from dipolar intermolecular force between molecules
$\delta H$ = The energy from hydrogen bonds between molecules

Likeness can then be determined using the *Ra* of the system and an interaction radius of the guest molecule (termed *Ri*) as shown below:

$$RED = \frac{Ra}{Ri}$$

where,

*RED* = Relative Energy Difference of the system
*Ra* = HSP Distance
*Ri* = *Ra* = HSP Distance

The RED between the encapsulating material (host) and water must be less than 1 for the encapsulating material (host) to dissolve in water. The RED between the flavour and water must be less than 1 for the flavour to dissolve in water; or the RED between the flavour and the encapsulating material (host) must be less than 1 for the flavour to be taken up by the encapsulating material (host).

## Claims

**1.** An aerosolisable formulation comprising

(i) water;
(ii) one or more flavours to be encapsulated;
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof; and
(iv) nicotine;

wherein in the presence of water the energy of binding of the one or more encapsulating materials with the one or more flavours to be encapsulated is from -0.5 to -8 kcal/mol.

**2.** An aerosolisable formulation comprising

(i) water
(ii) one or more flavours to be encapsulated; and
(iii) one or more encapsulating materials having a solubility in water of at least 50% of the solubility in water of the one or more flavours to be encapsulated, wherein the one or more encapsulating materials are selected from the group consisting of substituted ($\alpha$)-cyclodextrin, substituted ($\beta$)-cyclodextrin, substituted ($\gamma$)-cyclodextrin, and mixtures thereof; and
(iv) nicotine.

**3.** An aerosolisable formulation according to claim 2 wherein in the presence of water the energy of binding of the one or more encapsulating materials with the one or more flavours to be encapsulated is from -0.5 to -8 kcal/mol, such as from -2 to -8 kcal/mol, such as from -3 to -8 kcal/mol, such as from -4 to -7 kcal/mol.

**4.** An aerosolisable formulation according to any one of claims 1 to 3 wherein water is present in an amount of at least 75 wt.% based on the aerosolisable formulation, such as wherein water is present in an amount of at least 90 wt.% based on the aerosolisable formulation

5. An aerosolisable formulation according to any one of claims 1 to 4 wherein the nicotine is present in an amount of no greater than 1wt.% based on the aerosolisable formulation, such as wherein nicotine is present in an amount of from 0.01 to 0.6 wt.% based on the aerosolisable formulation.

6. An aerosolisable formulation according to any one of claims 1 to 5 further comprising at least one acid, such as wherein the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof, such as wherein the acid is selected from the group consisting of citric acid, benzoic acid, levulinic acid, sorbic acid, lactic acid, and mixtures thereof, such as wherein the acid is at least citric acid, and/or wherein the total content of acid present in the formulation is no greater than 1.0 mole equivalents based on the nicotine and/or wherein the total content of acid present in the solution is no less than 0.1 mole equivalents based on the nicotine.

7. An aerosolisable formulation according to any one of claims 1 to 6 wherein the one or more flavours are selected from (4-(para-)methoxyphenyl)-2-butanone, vanillin, γ-undecalactone, menthone, 5-propenyl guaethol, menthol, para-mentha-8-thiol-3-one and mixtures thereof, such as wherein the flavour is at least menthol, and/or wherein the one or more flavours are present in a total amount of no greater than 2 wt.% based on the aerosolisable formulation, such as wherein the one or more flavours are present in a total amount of from 0.01 to 1 wt.% based on the aerosolisable formulation.

8. An aerosolisable formulation according to claim 1 to 7 wherein the one more cyclodextrins is at least a substituted (β)-cyclodextrin.

9. An aerosolisable formulation according to any one of claims 1 to 8 wherein the one or more encapsulating materials are present in a total amount of no greater than 12 wt.% based on the aerosolisable formulation and/or wherein the one or more encapsulating materials have a solubility in water of at least 70 % of the solubility in water of the one or more flavours to be encapsulated, such as wherein the one or more encapsulating materials have a solubility in water of at least 90 % of the solubility in water of the one or more flavours to be encapsulated.

10. A process for forming an aerosol, the process comprising aerosolising an aerosolisable formulation comprising

(i) water;
(ii) one or more flavours to be encapsulated; and
(iii) one or more encapsulating materials selected from the group consisting of substituted (α)-cyclodextrin, substituted (β)-cyclodextrin, substituted (γ)-cyclodextrin, and mixtures thereof;

wherein in the presence of water the energy of binding of the one or more encapsulating materials with the one or more flavours to be encapsulated is from -0.5 to -8 kcal/mol.

11. A process according to claim 10 wherein the aerosol is formed by a process performed at a temperature below 50°C, such as wherein the aerosol is formed by applying ultrasonic energy to the aerosolised formulation.

12. A contained aerosolisable formulation comprising

(a) a container; and
(b) an aerosolisable formulation comprising

(i) water;
(ii) one or more flavours to be encapsulated;
(iii) one or more encapsulating materials selected from the group consisting of substituted (α)-cyclodextrin, substituted (β)-cyclodextrin, substituted (γ)-cyclodextrin, and mixtures thereof; and
(iv) nicotine;

wherein in the presence of water the energy of binding of the one or more encapsulating materials with the one or more flavours to be encapsulated is from -0.5 to -8 kcal/mol.

13. A contained aerosolisable formulation according to claim 12 wherein the container is configured for engagement with an electronic aerosol provision system.

**14.** An electronic aerosol provision system comprising:

(a) an aerosoliser for aerosolising formulation for inhalation by a user of the electronic aerosol provision system;
(b) a power supply comprising a cell or battery for supplying power to the aerosoliser
(c) an aerosolisable formulation comprising

(i) water;
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

**15.** A process for improving the sensory properties of an aerosolised formulation, the process comprising the steps of aerosolising an aerosolisable formulation comprising

(i) water;
(ii) one or more flavours to be encapsulated; and
(iii) one or more encapsulating materials selected from the group consisting of substituted ($\alpha$)-cyclodextrin, substituted ($\beta$)-cyclodextrin, substituted ($\gamma$)-cyclodextrin, and mixtures thereof;

wherein in the presence of water the energy of binding of the one or more encapsulating materials with the one or more flavours to be encapsulated is from -0.5 to -8 kcal/mol.

**Patentansprüche**

**1.** Aerosolisierbare Formulierung, umfassend

(i) Wasser;
(ii) einen oder mehrere zu verkapselnde Geschmacksstoffe;
(iii) ein oder mehrere Verkapselungsmaterialien ausgewählt aus der Gruppe bestehend aus substituiertem ($\alpha$)-Cyclodextrin, substituiertem ($\beta$)-Cyclodextrin, substituiertem ($\gamma$)-Cyclodextrin und Mischungen davon; und
(iv) Nicotin;

wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol beträgt.

**2.** Aerosolisierbare Formulierung, umfassend

(i) Wasser,
(ii) einen oder mehrere zu verkapselnde Geschmacksstoffe und
(iii) ein oder mehrere Verkapselungsmaterialien mit einer Löslichkeit in Wasser von mindestens 50 % der Löslichkeit in Wasser des einen oder der mehreren zu verkapselnden Geschmacksstoffe, wobei das eine oder die mehreren Verkapselungsmaterialien ein oder mehrere Cyclodextrine aus der Gruppe bestehend aus substituiertem ($\alpha$)-Cyclodextrin, substituiertem ($\beta$)-Cyclodextrin, substituiertem ($\gamma$)-Cyclodextrin und Mischungen davon ausgewählt sind; und
(iv) Nicotin.

**3.** Aerosolisierbare Formulierung nach Anspruch 2, wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol, wie -2 bis -8 kcal/mol, wie -3 bis -8 kcal/mol, wie -4 bis -7 kcal/mol, beträgt.

**4.** Aerosolisierbare Formulierung nach einem der Ansprüche 1 bis 3, wobei Wasser in einer Menge von mindestens 75 Gew.-%, bezogen auf die aerosolisierbare Formulierung, vorhanden ist, wobei Wasser beispielsweise in einer Menge von mindestens 90 Gew.-%, bezogen auf die aerosolisierbare Formulierung, vorhanden ist.

5. Aerosolisierbare Formulierung nach einem der Ansprüche 1 bis 4, wobei das Nicotin in einer Menge von nicht mehr als 1 Gew.-%, bezogen auf die aerosolisierbare Formulierung, vorhanden ist, wobei Nicotin beispielsweise in einer Menge von 0,01 bis 0,6 Gew.-%, bezogen auf die aerosolisierbare Formulierung, vorhanden ist.

6. Aerosolisierbare Formulierung nach einem der Ansprüche 1 bis 5, die ferner mindestens eine Säure umfasst, wobei die Säure aus der aus Essigsäure, Milchsäure, Ameisensäure, Citronensäure, Benzoesäure, Brenztraubensäure, Lävulinsäure, Bernsteinsäure, Weinsäure, Sorbinsäure, Propionsäure, Phenylessigsäure und Mischungen davon bestehenden Gruppe ausgewählt ist, wobei die Säure beispielsweise aus der aus Citronensäure, Benzoesäure, Lävulinsäure, Sorbinsäure, Milchsäure und Mischungen davon bestehenden Gruppe ausgewählt ist, wobei es sich bei der Säure mindestens um Citronensäure handelt, und/oder wobei der Gesamtgehalt der in der Formulierung vorhandenen Säure nicht mehr als 1,0 Moläquivalente, bezogen auf das Nicotin, beträgt und/oder wobei der Gesamtgehalt der in der Lösung vorhandenen Säure beispielsweise nicht weniger als 0,1 Moläquivalente, bezogen auf das Nicotin, beträgt.

7. Aerosolisierbare Formulierung nach einem der Ansprüche 1 bis 6, wobei der eine oder die mehreren Geschmacksstoffe aus (4-(para-)Methoxyphenyl)-2-butanon, Vanillin, γ-Undecalacton, Menthon, 5-Propenylguaethol, Menthol, para-Mentha-8-thiol-3-on und Mischungen davon ausgewählt sind, wobei der Geschmacksstoff beispielsweise mindestens Menthol ist, und/oder wobei der eine oder die mehreren Geschmacksstoffe in einer Gesamtmenge von nicht mehr als 2 Gew. %, bezogen auf die aerosolisierbare Formulierung, vorhanden sind, wobei der eine oder die mehreren Geschmacksstoffe beispielsweise in einer Gesamtmenge von 0,01 bis 1 Gew.-%, bezogen auf die aerosolisierbare Formulierung, vorhanden sind.

8. Aerosolierbare Formulierung nach Anspruch 1 bis 7, wobei es sich bei dem einen oder den mehreren Cyclodextrinen um mindestens ein substituiertes (β)-Cyclodextrin handelt.

9. Aerosolisierbare Formulierung nach einem der Ansprüche 1 bis 8, wobei der eine oder die mehreren Verkapselungsmaterialien in einer Gesamtmenge von nicht mehr als 12 Gew.-%, bezogen auf die aerosolisierbare Formulierung, vorhanden sind und/oder wobei das eine oder die mehreren Verkapselungsmaterialien eine Löslichkeit in Wasser von mindestens 70 % der Löslichkeit in Wasser von einem oder mehreren der zu verkapselnden Geschmacksstoffe in Wasser aufweisen, wobei das eine oder die mehreren Verkapselungsmaterialien beispielsweise eine Löslichkeit in Wasser von mindestens 90 % der Löslichkeit des einen oder der mehreren zu verkapselnden Geschmacksstoffe in Wasser aufweisen.

10. Verfahren zur Bildung eines Aerosols, wobei das Verfahren das Aerosolisieren einer aerosolisierbaren Formulierung umfasst, die Folgendes umfasst:

(i) Wasser;
(ii) einen oder mehrere zu verkapselnde Geschmacksstoffe und
(iii) ein oder mehrere Verkapselungsmaterialien ausgewählt aus der Gruppe bestehend aus substituiertem (α)-Cyclodextrin, substituiertem (β)-Cyclodextrin, substituiertem (γ)-Cyclodextrin und Mischungen davon;

wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol beträgt.

11. Verfahren nach Anspruch 10, wobei das Aerosol durch ein bei einer Temperatur unter 50 °C durchgeführtes Verfahren gebildet wird, wobei das Aerosol beispielsweise durch Anwendung von Ultraschallenergie auf die aerosolisierte Formulierung gebildet wird.

12. In einem Behälter befindliche aerosolisierbare Formulierung, umfassend:

(a) einen Behälter und
(b) eine aerosolisierbare Formulierung, umfassend

(i) Wasser;
(ii) einen oder mehrere zu verkapselnde Geschmacksstoffe;
(iii) ein oder mehrere Verkapselungsmaterialien ausgewählt aus der Gruppe bestehend aus substituiertem (α)-Cyclodextrin, substituiertem (β)-Cyclodextrin, substituiertem (γ)-Cyclodextrin und Mischungen davon; und

(iv) Nicotin;

wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol beträgt.

**13.** In einem Behälter befindliche aerosolisierbare Formulierung nach Anspruch 12, wobei der Behälter für die Kopplung mit einem elektronischen Aerosolbereitstellungssystem konfiguriert ist.

**14.** Elektronisches Aerosolbereitstellungssystem, umfassend:

(a) einen Vernebler für eine Aerosolformulierung zur Inhalation durch einen Benutzer des elektronischen Aerosolbereitstellungssystems;
(b) eine Stromversorgung, die eine Zelle oder eine Batterie zur Versorgung des Verneblers mit Strom umfasst;
(c) eine aerosolisierbare Formulierung, umfassend

(i) Wasser;
(ii) einen oder mehrere zu verkapselnde Geschmacksstoffe und
(iii) ein oder mehrere Verkapselungsmaterialien ausgewählt aus der Gruppe bestehend aus substituiertem ($\alpha$)-Cyclodextrin, substituiertem ($\beta$)-Cyclodextrin, substituiertem ($\gamma$)-Cyclodextrin und Mischungen davon;

wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol beträgt.

**15.** Verfahren zur Verbesserung der sensorischen Eigenschaften einer aerosolisierbaren Formulierung, wobei das Verfahren die Schritte des Aerosolisierens einer aerosolisierbaren Formulierung umfasst, die Folgendes umfasst:

(i) Wasser;
(ii) einen oder mehrere zu verkapselnde Geschmacksstoffe und
(iii) ein oder mehrere Verkapselungsmaterialien ausgewählt aus der Gruppe bestehend aus substituiertem ($\alpha$)-Cyclodextrin, substituiertem ($\beta$)-Cyclodextrin, substituiertem ($\gamma$)-Cyclodextrin und Mischungen davon;

wobei die Energie der Bindung des einen oder der mehreren Verkapselungsmaterialien mit dem einen oder den mehreren zu verkapselnden Geschmacksstoffen in Gegenwart von Wasser -0,5 bis -8 kcal/mol beträgt.

## Revendications

**1.** Formulation aérosolisable comprenant

(i) de l'eau ;
(ii) un ou plusieurs arômes à encapsuler ;
(iii) un ou plusieurs matériaux d'encapsulation choisis dans le groupe constitué par l'($\alpha$)-cyclodextrine substituée, la ($\beta$)-cyclodextrine substituée, la ($\gamma$)-cyclodextrine substituée et les mélanges de celles-ci ; et
(iv) de la nicotine ;

dans laquelle en présence d'eau, l'énergie de liaison du ou des matériaux d'encapsulation à l'arôme ou aux arômes à encapsuler va de -0,5 à -8 kcal/mol.

**2.** Formulation aérosolisable comprenant

(i) de l'eau
(ii) un ou plusieurs arômes à encapsuler ; et
(iii) un ou plusieurs matériaux d'encapsulation ayant une solubilité dans l'eau d'au moins 50 % de la solubilité dans l'eau du ou des arômes à encapsuler, dans laquelle le ou les matériaux d'encapsulation sont choisis dans le groupe constitué par l'($\alpha$)-cyclodextrine substituée, la ($\beta$)-cyclodextrine substituée, la ($\gamma$)-cyclodextrine substituée et les mélanges de celles-ci ; et
(iv) de la nicotine.

3. Formulation aérosolisable selon la revendication 2 dans laquelle en présence d'eau, l'énergie de liaison du ou des matériaux d'encapsulation à l'arôme ou aux arômes à encapsuler va de -0,5 à -8 kcal/mol, telle que de -2 à -8 kcal/mol, telle que de -3 à -8 kcal/mol, telle que de -4 à -7 kcal/mol.

4. Formulation aérosolisable selon l'une quelconque des revendications 1 à 3 dans laquelle l'eau est présente en une quantité d'au moins 75 % en poids sur la base de la formulation aérosolisable, telle que dans laquelle l'eau est présente en une quantité d'au moins 90 % en poids sur la base de la formulation aérosolisable.

5. Formulation aérosolisable selon l'une quelconque des revendications 1 à 4 dans laquelle la nicotine est présente en une quantité non supérieure à 1 % en poids sur la base de la formulation aérosolisable, telle que dans laquelle la nicotine est présente en une quantité allant de 0,01 à 0,6 % en poids sur la base de la formulation aérosolisable.

6. Formulation aérosolisable selon l'une quelconque des revendications 1 à 5 comprenant en outre au moins un acide, telle que dans laquelle l'acide est choisi dans le groupe constitué par l'acide acétique, l'acide lactique, l'acide formique, l'acide citrique, l'acide benzoïque, l'acide pyruvique, l'acide lévulinique, l'acide succinique, l'acide tartrique, l'acide sorbique, l'acide propionique, l'acide phénylacétique et les mélanges de ceux-ci, telle que dans laquelle l'acide est choisi dans le groupe constitué par l'acide citrique, l'acide benzoïque, l'acide lévulinique, l'acide sorbique, l'acide lactique et les mélanges de ceux-ci, telle que dans laquelle l'acide est au moins l'acide citrique et/ou dans laquelle la teneur totale d'acide présent dans la formulation n'est pas supérieure à 1,0 équivalent molaire sur la base de la nicotine et/ou dans laquelle la teneur totale d'acide présent dans la solution n'est pas inférieure à 0,1 équivalent molaire sur la base de la nicotine.

7. Formulation aérosolisable selon l'une quelconque des revendications 1 à 6 dans laquelle le ou les arômes sont choisis parmi la (4-(para-)méthoxyphényl)-2-butanone, la vanilline, la $\gamma$-undécalactone, la menthone, le 5-propénylguaétol, le menthol, la para-mentha-8-thiol-3-one et les mélanges de ceux-ci, telle que dans laquelle l'arôme est au moins le menthol et/ou dans laquelle le ou les arômes sont présents en une quantité totale non supérieure à 2 % en poids sur la base de la formulation aérosolisable, telle que dans laquelle le ou les arômes sont présents en une quantité totale allant de 0,01 à 1 % en poids sur la base de la formulation aérosolisable.

8. Formulation aérosolisable selon les revendications 1 à 7 dans laquelle la ou les cyclodextrines sont au moins une ($\beta$)-cyclodextrine substituée.

9. Formulation aérosolisable selon l'une quelconque des revendications 1 à 8 dans laquelle le ou les matériaux d'encapsulation sont présents en une quantité totale non supérieure à 12 % en poids sur la base de la formulation aérosolisable et/ou dans laquelle le ou les matériaux d'encapsulation ont une solubilité dans l'eau d'au moins 70 % de la solubilité dans l'eau du ou des arômes à encapsuler, telle que dans laquelle le ou les matériaux d'encapsulation ont une solubilité dans l'eau d'au moins 90 % de la solubilité dans l'eau du ou des arômes à encapsuler.

10. Procédé pour la formation d'un aérosol, le procédé comprenant l'aérosolisation d'une formulation aérosolisable comprenant

(i) de l'eau ;
(ii) un ou plusieurs arômes à encapsuler ; et
(iii) un ou plusieurs matériaux d'encapsulation choisis dans le groupe constitué par l'($\alpha$)-cyclodextrine substituée, la ($\beta$)-cyclodextrine substituée, la ($\gamma$)-cyclodextrine substituée et les mélanges de celles-ci ;

dans lequel en présence d'eau, l'énergie de liaison du ou des matériaux d'encapsulation à l'arôme ou aux arômes à encapsuler va de -0,5 à -8 kcal/mol.

11. Procédé selon la revendication 10 dans lequel l'aérosol est formé par un procédé réalisé à une température au-dessous de 50 °C, tel que dans lequel l'aérosol est formé par application d'énergie ultrasonique à la formulation aérosolisée.

12. Formulation aérosolisable contenue comprenant

(a) un récipient ; et
(b) une formulation aérosolisable comprenant

(i) de l'eau ;
(ii) un ou plusieurs arômes à encapsuler ;
(iii) un ou plusieurs matériaux d'encapsulation choisis dans le groupe constitué par l'($\alpha$)-cyclodextrine substituée, la ($\beta$)-cyclodextrine substituée, la ($\gamma$)-cyclodextrine substituée et les mélanges de celles-ci ; et
(iv) de la nicotine ;

dans laquelle en présence d'eau, l'énergie de liaison du ou des matériaux d'encapsulation à l'arôme ou aux arômes à encapsuler va de -0,5 à -8 kcal/mol.

**13.** Formulation aérosolisable contenue selon la revendication 12 dans laquelle le récipient est conçu pour venir en prise avec un système électronique de fourniture d'aérosol.

**14.** Système électronique de fourniture d'aérosol comprenant :

(a) un aérosoliseur pour l'aérosolisation d'une formulation pour inhalation par un utilisateur du système électronique de fourniture d'aérosol ;
(b) une alimentation électrique comprenant une pile ou une batterie pour l'apport d'énergie électrique à l'aérosoliseur
(c) une formulation aérosolisable comprenant

(i) de l'eau ;
(ii) un ou plusieurs arômes à encapsuler ; et
(iii) un ou plusieurs matériaux d'encapsulation choisis dans le groupe constitué par l'($\alpha$)-cyclodextrine substituée, la ($\beta$)-cyclodextrine substituée, la ($\gamma$)-cyclodextrine substituée et les mélanges de celles-ci ;

dans lequel en présence d'eau, l'énergie de liaison du ou des matériaux d'encapsulation à l'arôme ou aux arômes à encapsuler va de -0,5 à -8 kcal/mol.

**15.** Procédé pour l'amélioration des propriétés sensorielles d'une formulation aérosolisée, le procédé comprenant les étapes d'aérosolisation d'une formulation aérosolisable comprenant

(i) de l'eau ;
(ii) un ou plusieurs arômes à encapsuler ; et
(iii) un ou plusieurs matériaux d'encapsulation choisis dans le groupe constitué par l'($\alpha$)-cyclodextrine substituée, la ($\beta$)-cyclodextrine substituée, la ($\gamma$)-cyclodextrine substituée et les mélanges de celles-ci ;

dans lequel en présence d'eau, l'énergie de liaison du ou des matériaux d'encapsulation à l'arôme ou aux arômes à encapsuler va de -0,5 à -8 kcal/mol.

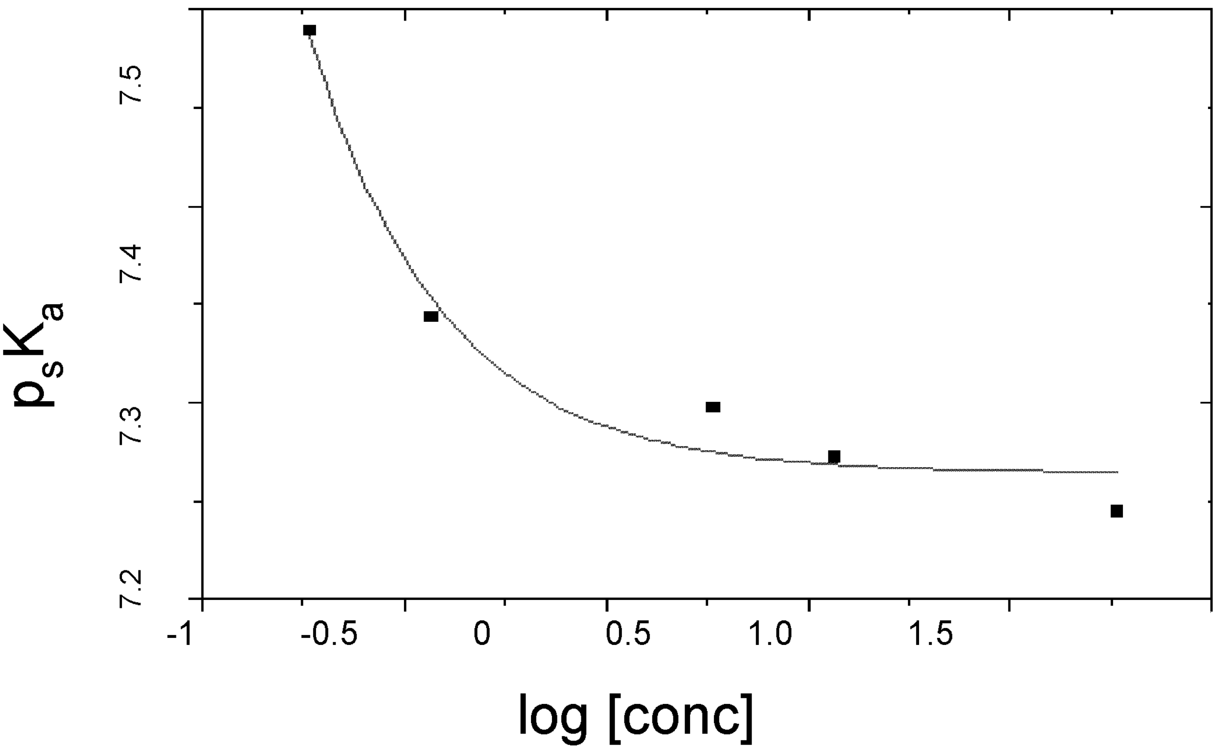


*Figure 1: Variation of* $p_sK_{a2}$ with nicotine concentration

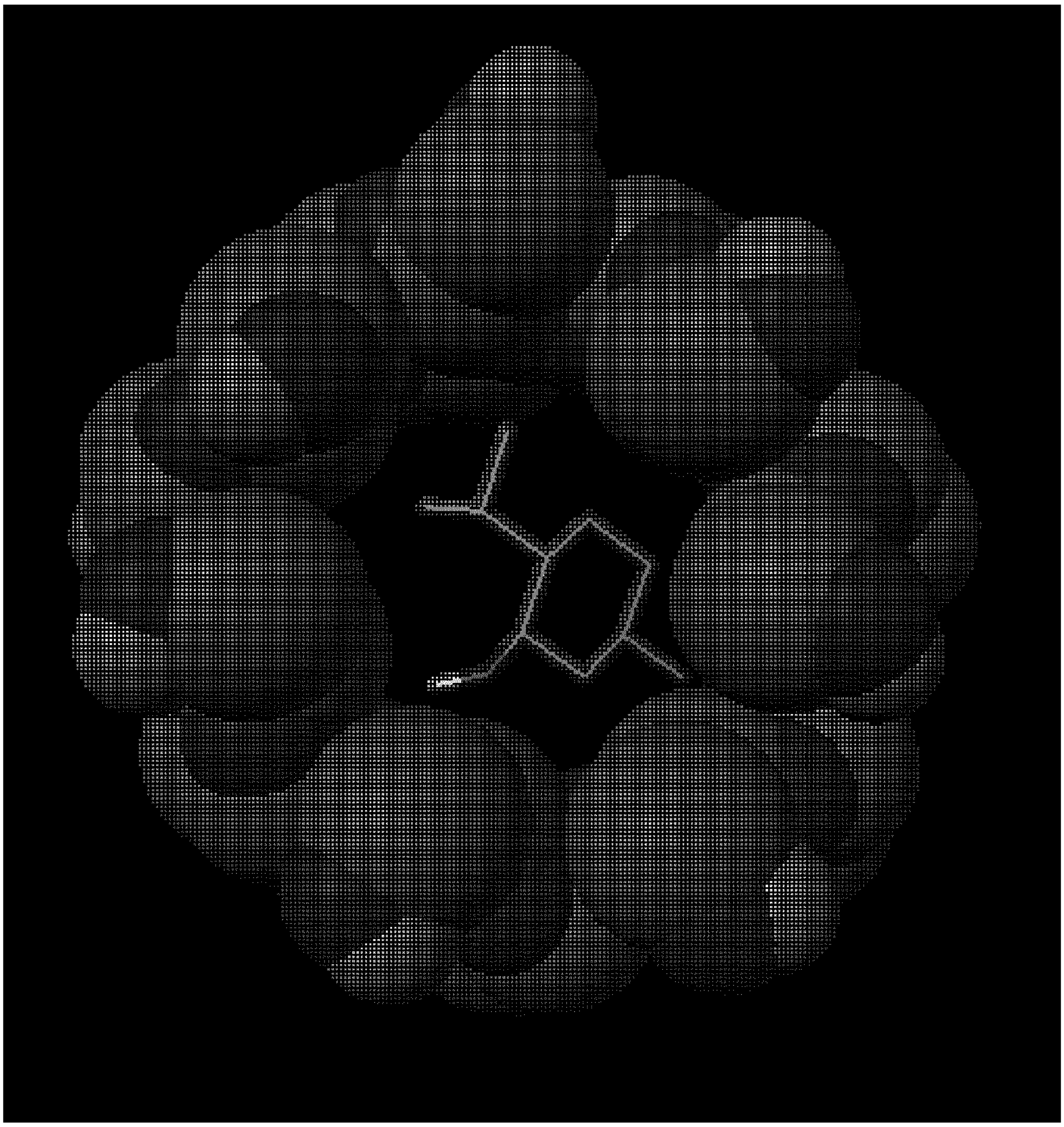

Figure 2 Docking schematic for unsubstituted beta CD

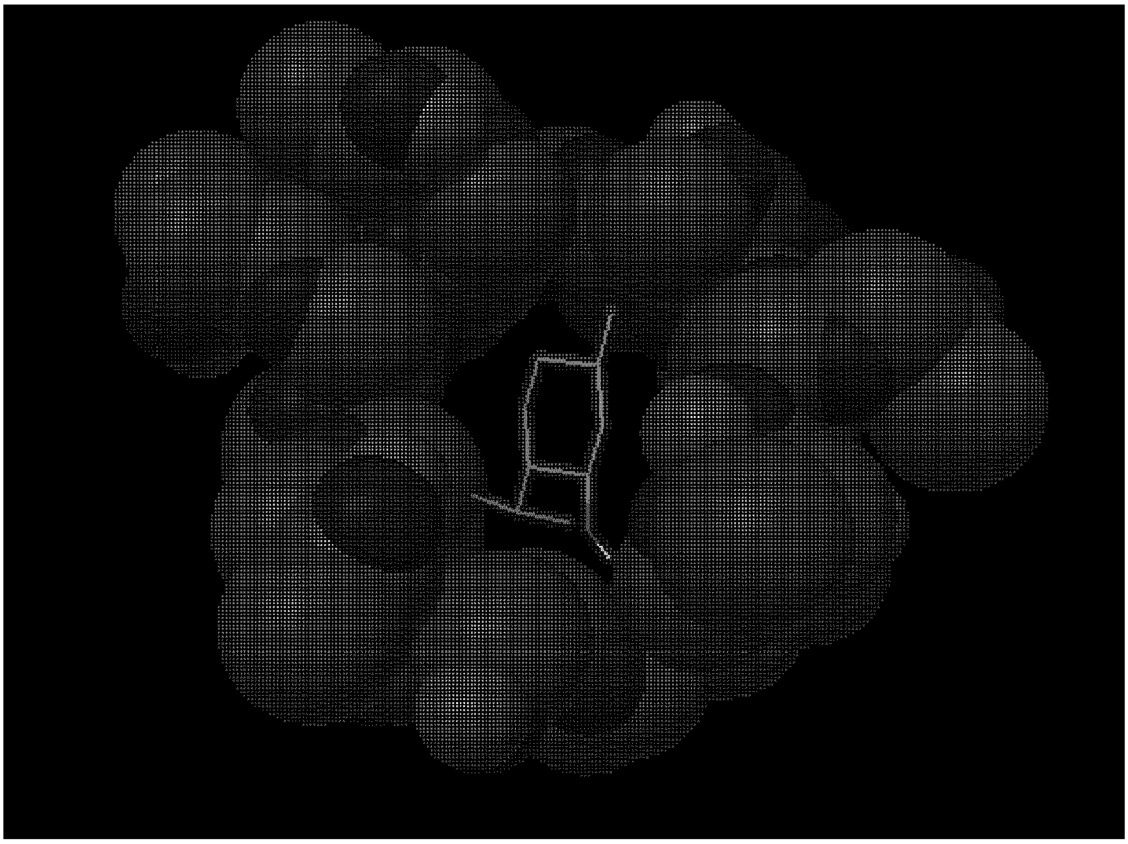

Figure 3 Docking schematic for HP-beta-CD with substitution at 7 sites

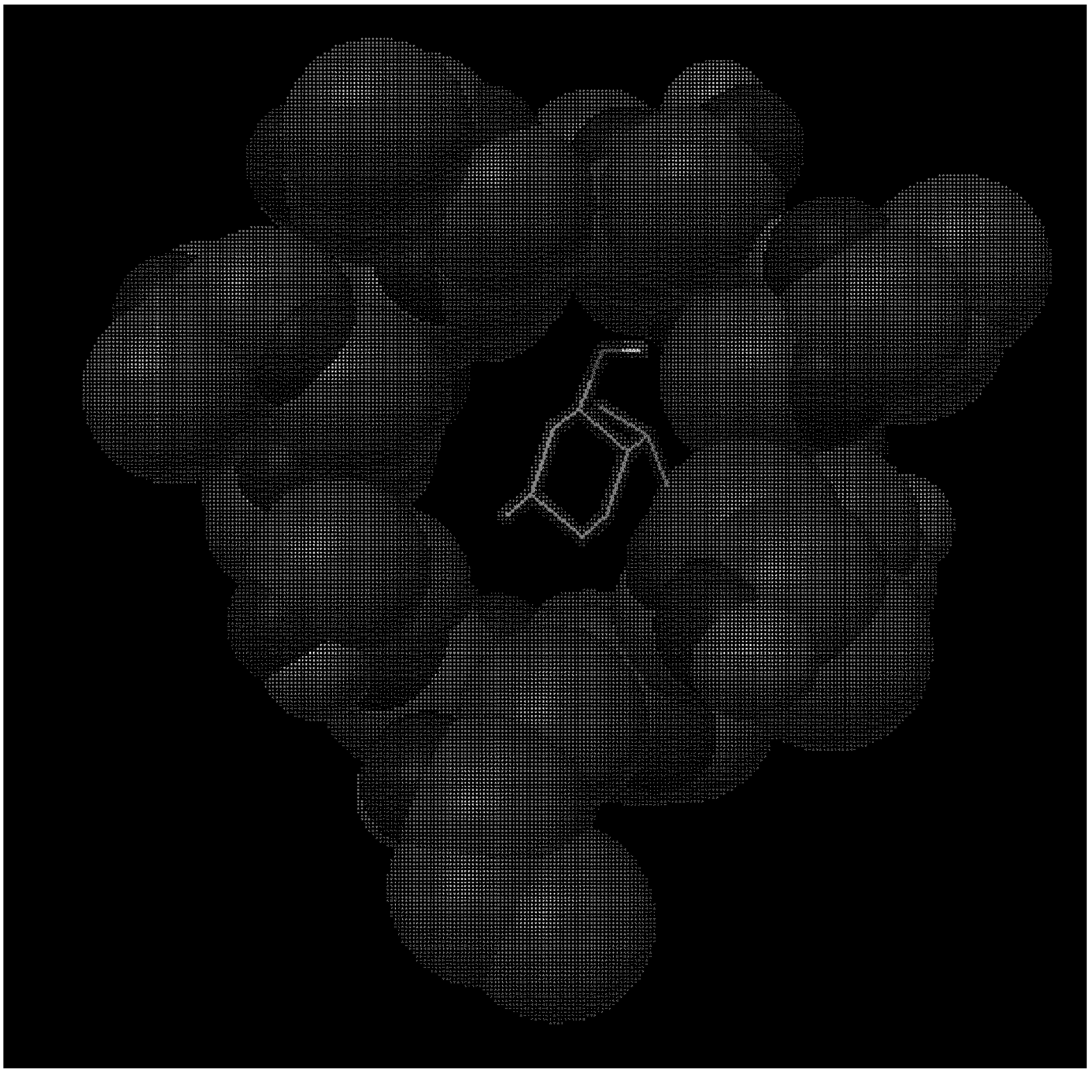

Figure 4 Docking schematic for HP-beta-CD with substitution at 5 sites

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016198759 A1


### Non-patent literature cited in the description


- **MORRIS, G. M** ; **HUEY, R.** ; **LINDSTROM, W.** ; **SANNER, M. F** ; **BELEW, R. K.** ; **GOODSELL, D. S.** ; **OLSON, A. J.** Autodock4 and AutodockTools4: automated docking with selective receptor flexibility. *J. Computational Chemistry*, 2009, 2785-2791
- **PETER M. CLAYTON** ; **CARL A. VAS** ; **TAM T. T. BUI** ; **ALEX F. DRAKE** ; **KEVIN MCADAM**. Spectroscopic investigations into the acid-base properties of nicotine at different temperatures. *Anal. Methods*, 2013, vol. 5, 81-88
- BRITISH PHARMACOPOEIA COMMISSION. *British Pharmacopoeia. London, England: Stationery Office and COUNCIL OF EUROPE*, 2013